(19) Europäisches Patentamt
European Patent Office
Office européen des brevets

(11) Veröffentlichungsnummer: **0 006 218**
**B1**

(12) **EUROPÄISCHE PATENTSCHRIFT**

(45) Veröffentlichungstag der Patentschrift: **26.05.82**

(21) Anmeldenummer: **79101912.8**

(22) Anmeldetag: **12.06.79**

(51) Int. Cl.³: **C 07 C 103/48,**
C 07 C 103/84,
C 07 C 103/66,
A 61 K 31/195

(54) **Substituierte Aminosäuren, ihre Verwendung und Herstellung und sie enthaltende Arzneimittel.**

(30) Priorität: **14.06.78 CH 6504/78**

(43) Veröffentlichungstag der Anmeldung:
**09.01.80 Patentblatt 80/1**

(45) Bekanntmachung des Hinweises auf die Patenterteilung:
**26.05.82 Patentblatt 82/21**

(84) Benannte Vertragsstaaten:
**AT BE CH DE FR GB IT LU NL SE**

(56) Entgegenhaltungen:
**DE - A - 1 917 036**
**FR - A - 2 100 836**

(73) Patentinhaber: **Byk Gulden Lomberg Chemische Fabrik GmbH**
**Byk-Gulden-Strasse 2**
**D-7750 Konstanz (DE)**

(72) Erfinder: **Krastinat, Walter, Dr.**
**Erfurter Strasse 9**
**D-7750 Konstanz (DE)**
Erfinder: **Riedel, Richard, Dr.**
**Bruelstrasse 22**
**D-7750 Konstanz (DE)**
Erfinder: **Wolf, Horst, Dr.**
**Brandesstrasse 29**
**D-7750 Konstanz (DE)**

**EP 0 006 218 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Substituierte Aminosäuren, ihre Verwendung und Herstellung und sie enthaltende Arzneimittel

Die Erfindung betrifft substituierte Aminosäuren, ihre Verwendung und Herstellung und sie enthaltende Arzneimittel.

Die erfindungsgemäßen Verbindungen werden in der pharmazeutischen Industrie zur Herstellung von Medikamenten verwendet.

Bei der Untersuchung von N-Benzoyl-anilinoalkancarbonsäuren [D. Evans et al., J. Med. Chem. 12(1969)1006—10] wiesen die entsprechenden Buttersäuren keine entzündungshemmende Wirkung auf, während in der deutschen Offenlegungsschrift DE—OS 19 17 036 choleretisch wirkende N-Acylanilinoalkansäuren beschrieben werden, denen auch noch weitere Wirkungen zugesprochen werden (DE—OS 24 50 680). $\alpha$-Phenyl-benzyliden-$\omega$-amino-alkansäuren und ihre Derivate sollen in antiepileptisch wirkenden Mitteln eingesetzt werden (DE—OS 26 34 288). In einer Reihe von deutschen Offenlegungsschriften (DE—OS 21 31 626, 21 31 674, 21 31 675, 21 31 679 und 21 31 680) werden Trialkoxybenzoylaminoalkancarbonsäuren beschrieben, die zur Prophylaxe und Behandlung des Herzinfarkts eingesetzt werden sollen. Es wurde nun eine neue Klasse von Acylbiphenylylaminoalkansäuren synthetisiert, die weder in den erwähnten Publikationen genannt, noch durch sie nahegelegt wird. Ferner wurde gefunden, daß diese Acylbiphenylylaminoalkansäuren interessante, besonders vorteilhafte pharmakologische Eigenschaften aufweisen.

Gegenstand der Erfindung sind Acylbiphenylylaminoalkansäuren der allgemeinen Formel I

$$R^1\text{—CO—}\underset{\displaystyle A}{\underset{|}{N}}\text{—}C_nH_{2n}\text{—COOH} \qquad (I),$$

worin

$R^1$ einen aliphatischen oder alicyclischen Kohlenwasserstoffrest oder einen Phenylrest,

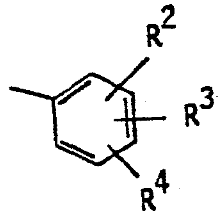

A einen gegebenenfalls hydrierten Biphenylylrest, der durch Halogenatome, Alkyl- oder Alkoxygruppen mit 1 bis 4 Kohlenstoffatomen, gegebenenfalls substituierte Aminogruppen, Hydroxy- oder Nitrogruppen substituiert sein kann,

n eine positive ganze Zahl von 3 bis 5 bedeuten,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe bedeuten, und ihre Salze mit anorganischen oder organischen Basen.

Als aliphatische Kohlenwasserstoffreste, die gesättigte oder ungesättigt sein können, kommen geradkettige oder verzweigte Alkylreste mit 1 bis 7 Kohlenstoffatomen in Frage. Geradkettige Alkylreste sind der Methyl-, Ethyl-, Propyl-, Butyl-, Pentyl, Hexyl- oder Heptylrest, von denen die mit 1 bis 5, vor allem mit 1 bis 3, Kohlenstoffatomen bevorzugt sind. Verzweigte Alkylreste mit 3 bis 7 Kohlenstoffatomen sind z.B. der Isopropyl-, Isobutyl-, sek.-Butyl- oder tert.-Butylrest, von denen die mit 3 bis 5, vor allem mit 3 Kohlenstoffatomen bevorzugt sind. Ungesättigte Kohlenwasserstoffreste sind Alkenyl- und Alkinylrest mit 2 bis 7 Kohlenstoffatomen, beispielsweise der Ethenyl-, der Ethinyl-, der 1-Propenyl-, 1,3-Butadienyl-, 2-Butinylrest, von denen der 1-Propenylrest bevorzugt ist. Als alicyclische Kohlenwasserstoffreste kommen Cycloalkylreste mit 3 bis 10 Kohlenstoffatomen, beispielsweise der Cyclopropyl-, Cyclobutyl-, Cyclopentyl-, Cyclohexyl-, Cycloheptyl- oder Cyclooctylrest, von denen die mit 5 bis 7 Kohlenstoffen bevorzugt sind, in Frage.

Als Halogenatome $R^2$, $R^3$ und $R^4$ kommen Fluor, Chlor und Brom, bevorzugt Fluor und Chlor, insbesondere Chlor, in Betracht. Als Alkylgruppen bzw. Alkoxygruppen $R^2$, $R^3$ und $R^4$ seien unter anderem die mit 1 bis 4 Kohlenstoffatomen genannt, von denen die mit 1 bis 3, vor allem die mit 1 Kohlenstoffatom(en) bevorzugt sind. Als Acyloxygruppen kommen unter anderem —O—CO—$R^1$-Gruppen, in denen $R^1$ die vorstehend angegebene Bedeutung hat, in Betracht, von denen die Alkanoyl-oxygruppe mit 1 bis 7, insbesondere mit 2 bis 5, Kohlenstoffatomen, vor allem die Acetoxygruppe, bevorzugt sind. Neben der unsubstituierten Aminogruppe kommen als Substituenten $R^2$, $R^3$ und $R^4$ vorwiegend substituierte Aminogruppen in Frage, von denen beispielsweise genannt seien Alkylamino- und Dialkylaminogruppen mit 1 bis 4, vorzugsweise 1 oder 2 Kohlenstoffatomen im Alkylrest sowie Acylaminogruppen

mit den üblichen zum Schutz von Aminogruppen verwendeten Acylgruppen, wie Alkanoylgruppen mit 2 bis 5 Kohlenstoffatomen.

Als Biphenylylreste kommen der 2-, 3- oder 4-Biphenylylrest in Frage. Partiell hydrierte Biphenylylreste sind beispielsweise der 4-Cyclohexylphenylrest, der 4-Phenylcyclohexylrest, der 2-Cyclohexylphenylrest. Halogenatome als Substituenten am Biphenylylrest sind z.B. Fluor, Chlor oder Brom, vorzugsweise Chlor, Alkyl- oder Alkoxygruppen als Substituenten am Biphenylylrest haben vorzugsweise ein Kohlenstoffatom.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin, Chinolin, genannt.

Eine Ausgestaltung der Erfindung sind Acylbiphenylylaminoalkansäuren der allgemeinen Formel I*

$$R^{1*}—CO—\underset{\displaystyle A^*}{\underset{|}{N}}—C_{n*}H_{2n*}—COOH \qquad (I^*),$$

worin

$R^{1*}$ einen aliphatischen Kohlenwasserstoffrest mit 1 bis 7 Kohlenstoffatomen, einen alicyclischen Kohlenwasserstoffrest mit 3 bis 10 Kohlenstoffatomen oder einen Phenylrest

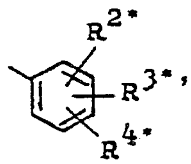

A* eine Gruppe der Formel

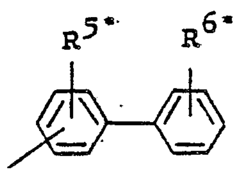 , 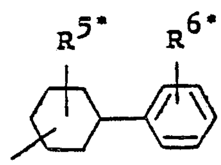 oder 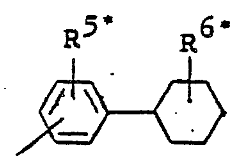

n* eine positive ganze Zahl von 3 bis 5 bedeuten,

$R^{2*}$, $R^{3*}$ und $R^{4*}$ gleich oder verschieden sind und ein Wasserstoffatom ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe oder eine Trifluormethylgruppe bedeuten,

$R^{5*}$ und $R^{6*}$ gleich oder verschieden sind und ein Wasserstoffatom, ein Halogenatom, eine Methylgruppe, eine Methoxygruppe, eine Hydroxygruppe oder eine Nitrogruppe, bedeuten, und ihre Salze mit anorganischen oder organischen Basen.

Eine andere Ausgestaltung der Erfindung sind Acylbiphenylylaminoalkansäuren der allgemeinen Formel I**

$$R^{1**}—CO—\underset{\displaystyle A^{**}}{\underset{|}{N}}—C_{n**}H_{2n**}—COOH \qquad (I^{**}),$$

worin

$R^{1**}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylrest

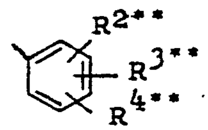

$A^{**}$ eine Gruppe der Formel

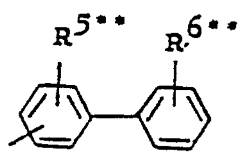 oder 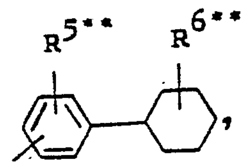 ,

$n^{**}$ eine positive ganze Zahl von 3 bis 5,
$R^{2**}$ ein Wasserstoffatom bedeuten,
$R^{3**}$ und $R^{4**}$ gleich oder verschieden sind und ein Wasserstoffatom, ein Halogenatom, eine Hydroxygruppe, eine Methoxygruppe, eine Methylgruppe, eine Alkanoyloxygruppe mit 2 bis 5 Kohlenstoffatomen, eine Nitrogruppe oder eine Trifluormethylgruppe bedeuten,
einer der Substituenten $R^{5**}$ oder $R^{6**}$ ein Wasserstoffatom und der andere ein Wasserstoffatom, ein Halogenatom, eine Methylgruppe, eine Methoxygruppe, eine Hydroxygruppe oder eine Nitrogruppe bedeutet, und ihre Salze mit anorganischen oder organischen Basen.

Eine weitere Ausgestaltung der Erfindung sind Acylbiphenylylaminoalkansäuren der allgemeinen Formel $I^{***}$

$$R^{1***}\text{—CO—}\underset{\displaystyle A^{***}}{\underset{|}{N}}\text{—}C_{n^{***}}H_{2n^{***}}\text{—COOH} \qquad (I^{***}),$$

worin
$R^{1***}$ einen aliphatischen Kohlenwasserstoffrest mit 1 bis 3 Kohlenstoffatomen oder einen Phenylrest

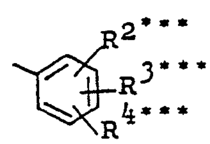 ,

$A^{***}$ eine Gruppe der Formel

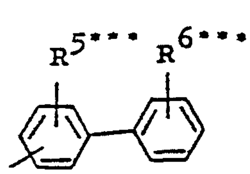 oder 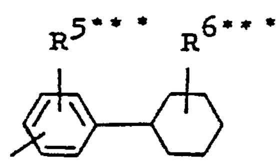

$n^{***}$ eine positive ganze von 3 bis 5,
$R^{2***}$ ein Wasserstoffatom bedeuten,
$R^{3***}$ und $R^{4***}$ gleich oder verschieden sind und ein Wasserstoffatom, ein Fluoratom, ein Chloratom, eine Hydroxygruppe, eine Methoxygruppe oder eine Trifluormethylgruppe bedeuten,
einer der Substituenten $R^{5***}$ oder $R^{6***}$ ein Wasserstoffatom und der andere ein Wasserstoffatom oder eine Methoxygruppe bedeutet, und ihre Salze mit anorganischen oder anorganischen Basen.

Eine bevorzugte Ausgestaltung der Erfindung sind Acylbiphenylylaminoalkansäuren der allgemeinen Formel $I^{****}$

$$R^{1****}\text{—CO—}\underset{\displaystyle A^{****}}{\underset{|}{N}}\text{—}C_{n^{****}}H_{2n^{****}}\text{—COOH} \qquad (I^{****}),$$

worin
$R^{1****}$ eine Alkylgruppe mit 1 bis 3 Kohlenstoffatomen, einen Alkenylrest mit 2 bis 3 Kohlenstoffatomen oder eine Phenylgruppe

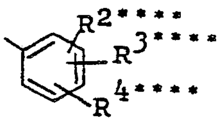

A**** eine Gruppe der Formel

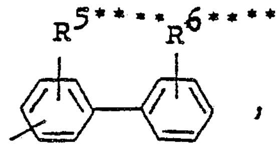

,

n**** eine positive ganze Zahl von 3 bis 5,
R²**** ein Wasserstoffatom,
R³**** ein Wasserstoffatom, ein Fluoratom, ein Chloratom, eine Hydroxygruppe, eine Methoxygruppe oder Trifluormethylgruppe,
R⁴**** ein Wasserstoffatom oder ein Chloratom,
einer der Substituenten R⁵**** oder R⁶**** ein Wasserstoffatom und der andere ein Wasserstoffatom oder eine Methoxygruppe bedeutet, und ihre pharmakologisch verträglichen Salze mit anorganischen und organischen Basen.

Besonders bevorzugte Vertreter der Ausgestaltung I**** sind solche, in denen R³**** ein Fluoratom, ein Chloratom, eine Hydroxygruppe oder eine Methoxygruppe bedeutet, R⁵**** und R⁶**** Wasserstoffatome darstellen, A****, R¹****, n****, R²**** und R⁴**** die oben angegebenen Bedeutungen haben, sowie ihre pharmakologisch verträglichen Salze mit anorganischen und organischen Basen.

Weitere besonders bevorzugte Vertreter der Ausgestaltungen I*** bzw. I**** sind solche, in denen A*** bzw. A**** die Bedeutung eines 2-Biphenylylrestes hat, und ihre pharmakologisch verträglichen Salze mit anorganischen und organischen Basen.

Als durch die allgemeine Formel I erfaßte Verbindungen seien beispielsweise genannt
4-[4-Methoxy-N-(2'-fluor-biphenyl-2-yl)-benzamido]-buttersäure,
4-[2,4-Dichlor-N-(6-methyl-biphenyl-2-yl)-benzamido]-buttersäure,
4-[N-(2'-Ethyl-biphenyl-2-yl)-n-butyramido]-buttersäure,
4-[3-Fluor-4-methyl-N-(3,2-dimethyl-biphenyl-2-yl)-benzamido]-buttersäure,
4-[3,5-Dimethoxy-N-(4-chlor-biphenyl-3-yl)-benzamido]-buttersäure,
4-[N-(4,4'-Dimethyl-biphenyl-3-yl)-hexanoylamido]-buttersäure,
4-[3-Trifluormethyl-N-(6-ethoxy-biphenyl-3-yl)-benzamido]-buttersäure,
4-[2-Brom-N-(2'-methoxy-biphenyl-4-yl)-benzamido]-buttersäure,
4-[3-Methoxy-4-methyl-N-(4'-methoxy-biphenyl-4-yl)-benzamido]-buttersäure,
5-[4-Methyl-3-nitro-N-(biphenyl-2-yl)-benzamido]-valeriansäure,
5-[3-Chlor-N-(4'-methoxy-biphenyl-4-yl)-benzamido]-valeriansäure,
5-[4-Fluor-N-(6-ethoxy-biphenyl-3-yl)-benzamido]-valeriansäure,
5-[4-Methoxy-N-(2'-fluor-biphenyl-2-yl)-benzamido]-valeriansäure,
5-[N-(4'-Chlor-biphenyl-4-yl)-methacryloylamido]-valeriansäure,
6-[2-Methoxy-N-(6-methyl-biphenyl-2-yl)-benzamido]-capronsäure,
6-[3,5-Dichlor-N-(4,4'-dimethyl-biphenyl-3-yl)-benzamido]-capronsäure,
6-[2,4-Dimethyl-N-(4'-chlor-biphenyl-4-yl)-benzamido]-capronsäure,
6-[3-Methoxy-4-methyl-N-(2'-fluor-biphenyl-2-yl)-benzamido]-capronsäure,
6-[4-Nitro-N-(biphenyl-2-yl)-benzamido]-capronsäure,
6-[N-(4'-Ethoxy-biphenyl-4-yl)-isovaleroylamido]-capronsäure,

Bevorzugte Vertreter der erfindungsgemäßen Verbindungen sind
4-[4-Chlor-N-(biphenyl-2-yl)-benzamido]-buttersäure,
4-[N-(Biphenyl-2-yl)-acetamido]-buttersäure,
4-[4-Fluor-N-(biphenyl-2-yl)-benzamido]-buttersäure,
4-[N-(Biphenyl-2-yl)-crotonoylamido]-buttersäure,
6-[2,4-Dichlor-N-(biphenyl-2-yl)-benzamido]-capronsäure,
6-[5-Chlor-2-methoxy-N-(biphenyl-2-yl)-benzamido]-capronsäure,
5-[2-Hydroxy-N-(biphenyl-2-yl)-benzamido]-valeriansäure,
6-[4-Chlor-N-(biphenyl-2-yl)-benzamido]-capronsäure
und ihre Salze.

Die erfindungsgemäß Verbindungen weisen wertvolle pharmakaologische Eigenschaften auf, die

sie gewerblich verwertbar machen. Sie entfalten an Warmblütlern eine Schutzwirkung für Magen und Leber, daneben bewirken sie eine Steigerung der Sekretion von Pankreas und Leber (Galle). Außerdem bewirken sie eine Hemmung der Glukosebildung aus Lactat und Pyruvat in der Leber.

Aufgrund ihrer vorteilhaften Wirksamkeit sind die Acylbiphenylaminoalkansäuren zur Behandlung und Prophylaxe von Krankheiten, die auf Erkrankungen des Magens oder Darms oder auf Minderleistungen von Pankreas, Galle und/oder Leber beruhen, geeignet. Beispielsweise werden bahandelt Magen- oder Darmulcera, Billroth II, Pankreas-Insuffizienz, Sprue, Maldigestionen und Malabsorptionen verschiedener Genese, akute und chronische Pankreatitis, indirekte Störungen der Pankreas-Funktion (Unterstützung der Sekretin- und Pankreozymin-Produktion), daneben Gallenblasen- und Gallenwegentzündungen, Gallenflußstörungen, Motilitätsstörungen der Gallenwege, Völlegefühl, Blähungen, Obstipationen, Oberbauchbeschwerden, hepato-biläre Funktionsstörungen, akute und chronische Hepatitis, Leberintoxikationen, Fettleber, Diabetes (maturity onset diabetes), Insulinmangeldiabetes in der Form des "brittle diabetes", diabetische Spätschäden.

Gegenstand der Erfindung sind außerdem die erfindungsgemäßen Verbindungen zur Verwendung bei der Bekämpfung der oben angegebenene Krankheiten.

Ein weiterer Gegenstand der Erfindung sind Arzneimittel, die eine oder mehrere der Acylbiphenylylaminoalkansäuren der allgemeinen Formel I

$$R^1—CO—\underset{\displaystyle A}{\underset{|}{N}}—C_nH_{2n}—COOH \qquad (I),$$

worin

$R^1$ einen aliphatischen oder alicyclischen Kohlenwasserstoffrest oder einen Phenylrest,

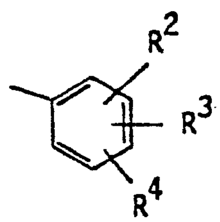

A einen gegebenenfalls hydrierten Biphenylylrest, der durch Halogenatome, Alkyl- oder Alkoxygruppen mit 1 bis 4 Kohlenstoffatomen, gegebenenfalls substituierte Aminogruppen, Hydroxy- oder Nitrogruppen substituiert sein kann,

n eine positive ganze Zahl von 3 bis 5 bedeuten,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe bedeuten, und/oder ihre pharmakologisch verträglichen Salze mit anorganischen oder organischen Basen enthalten.

Ausgestaltungen der Arzneimittel sind solche, die Acylbiphenylylaminoalkansäuren der Formeln I*, I**, I***, I**** oder ihre bevorzugten Vertreter und/oder ihre pharmakologisch verträglichen Salze mit anorganischen oder organischen Basen enthalten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

In Übereinstimmung mit der Erfindung können im human- und veterinärmedizinischen Bereich die Wirkstoffe in jeder beliebigen Form, z.B. systemisch, angewandt werden unter der Voraussetzung, daß die Ausbildung bzw. Aufrechterhaltung von ausreichenden Blut- oder Gewebsspiegeln oder Lokalkonzentrationen an Wirkstoffen gewährleistet ist. Das kann entweder durch orale, rektale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion, einer Suspension, eines Sols oder eines Gels sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein

Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung enthalten, wenn sie in Einheitsdosen vorliegen und für die Applikation, z.B. am Menschen bestimmt sind, etwa 0,5 bis 1000 mg, vorteilhafterweise 1 bis 750 mg und insbesondere 5 bis 500 mg Wirkstoff.

Im allgemeinen hat es sich sowohl in der Human- als auch in der Veterinärmedizin als vorteilhaft erwiesen, den oder die Wirkstoffe bei oraler Gabe in einer Tagesdosis von etwa 0,01 bis etwa 40, vorzugsweise 0,1 bis 30, insbesondere 0,2 bis 20 mg/kg Körpergewicht, gegebenenfalls in Form mehrerer, vorzugsweise 2 bis 3 Einzelgaben zur Erzielung der gewünschten Ergebnisse zu verabreichen. Eine Einzelgabe enthält den oder die Wirkstoffe in Mengen von etwa 0,01 bis etwa 20, vorzugsweise 0,1 bis 15, insbesondere 0,2 bis 10 mg/kg Körpergewicht.

Bei einer parenteralen Behandlung, z.B. einer intramuskulären oder intravenösen Applikation, können ähnliche Dosierungen zur Anwendung kommen. Bei dieser Therapie werden etwa 50 bis 1000 mg Wirkstoff appliziert.

Die therapeutische Verabreichung der pharmazeutischen Zubereitung erfolgt bei Dauermedikation im allgemeinen zu festgelegten Zeitpunkten, wie 1 bis 4 mal am Tage, z.B. jeweils vor oder nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann der Fachmann aufgrund seines Fachwissens jederzeit vornehmen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzuges, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen. Die Trägerstoffe werden jeweils vom Fachmann auf die mit den pharmazeutischen Zubereitungen zu behandelnden Krankheiten abgestimmt.

Zur oralen Anwendung können z.B. Tabletten, Dragées, harte und weiche Kapseln, z.B. aus Gelatine, dispergierbare Pulver; Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt und damit z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Natriumcyclamat, Saccharin, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den obengenannten, sowie mit Süssungsmitteln, Geschmacksmitteln und Farbstoffen enthalten.

Emulsionen können z.B. Oliven-, Erdnuß oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe gelangen Suppositorien, die mit Hilfe von bei Rektaltemperatur schmelzenden Bindemitteln, beispielsweise Kakaobutter oder Polyethylenglycolen, hergestellt werden.

Zur parenteralen Anwendung der Arzneistoffe dienen steril injizierbare wäßrige Suspensionen,

isotonische Salzlösungen oder sonstige Lösungen, die Dispergier- oder Benetzungsmittel und/oder pharmakologisch verträgliche Verdünnungsmittel, z.B. Propylen- oder Butylenglycol, enthalten können.

Der oder die Wirkstoffe können gegebenenfalls mit einem oder mehreren der angegebenen Träger- oder Zusatzstoffe auch in mikroverkapselter Form formuliert werden.

Sollen die erfindungsgemäßen Acylbiphenylylaminoalkansäuren und/oder ihre Salze zur Behandlung von Krankheiten, die auf Erkrankungen des Magens oder Darms oder auf Minderleistungen von Pankreas, Galle und/oder Leber beruhen, eingesetzt werden, so können die pharmazeutischen Zubereitungen auch einen oder mehrere andere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Entschäumungsmittel, beispielweise Dimethylpolysiloxan; Laxantien, beispielsweise Bisacodyl; Quellmittel; gegebenenfalls auch Fermente, Gallensäuren, Antibiotika, Vitamine, Aminosäuren, Fettsäuregemische etc. enthalten.

Sollen die erfindungsgemäßen Acylbiphenylylaminoalkansäuren und/oder ihre Salze als Antidiabetika formuliert werden, so können die pharmazeutischen Zubereitungen weiterhin einen oder mehrere pharmakologisch aktive Bestandteile anderer Arzneimittelgruppen, wie zusätzliche Antidiabetica (Sulfonamide, Sulfonylharnstoffe u.a.), z.B. Carbutamid, Tolbutamid, Chlorpropamid, Glibenclamid, Glibornurid, Glisoxepid, Gliquidon, Glymidin, oder Hypolipidämika, wie Bezafibrat oder Nikotinsäure sowie deren Derivate und Salze enthalten.

Ein weiterer Gegenstand der Erfindung ist ein Verfahren zur Herstellung der Acylbiphenylylaminoalkansäuren der allgemeinen Formel I

$$R^1—CO—\underset{\displaystyle A}{\underset{|}{N}}—C_nH_{2n}—COOH \qquad (I),$$

worin

$R^1$ einen aliphatischen oder alicyclischen Kohlenwasserstoffrest oder einen Phenylrest,

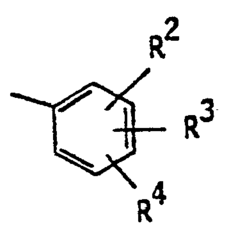

A einen gegebenenfalls hydrierten Biphenylylrest, der durch Halogenatome, Alkyl- oder Alkoxygruppen mit 1 bis 4 Kohlenstoffatomen, gegebenenfalls substituierte Aminogruppen, Hydroxy- oder Nitrogruppen substituiert sein kann,

n eine positive ganze Zahl von 3 bis 5 Bedeuten,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe eine Nitrogruppe oder eine Trifluoromethylgruppe bedeuten, sowie ihrer Salze mit anorganischen und organischen Basen, das dadurch gekennzeichnet ist, daß man

a) eine Biphenylylaminoalkansäure der allgemeinen Formel II

$$A—NH—C_nH_{2n}—COOH \qquad (II),$$

worin A und n die vorstehend angegebene Bedeutung haben, gegebenenfalls unter Schutz der Carboxylgruppe, mit einem Acylderivat der allgemeinen Formel III

$$R^1—CO—R^7 \qquad (III),$$

worin $R^7$ eine Fluchtgruppe oder eine $R^1—CO—O$-Gruppe darstellt und $R^1$ die oben angegebene Bedeutung hat, acyliert und gewünschtenfalls anschließend in die Salze überfuhrt oder

b) eine Biphenylylaminoalkensäure der allgemeinen Formel IV

$$R^1—CO—\underset{\displaystyle A}{\underset{|}{N}}—C_nH_{2n-2}—COOH \qquad (IV)$$

worin $R^1$, A und n die vorstehend angegebene Bedeutung haben, gegebenenfalls unter Schutz der

Carboxylgruppe hydriert und gewünschtenfalls anschließend in die Salze überführt oder

c) ein funktionelles Acylbiphenylylaminoalkansäurederivat der allgemeinen Formel V

$$R^1—CO—N(A)—C_nH_{2n}—G \quad (V),$$

worin $R^1$, A und n die oben angegebene Bedeutung haben und G ein funktionelles Derivat einer Carboxylgruppe bedeutet, solvolysiert und gewünschtenfalls anschließend in die Salze überführt.

Zur Herstellung der Verbindungen der Ausgestaltungen I*, I**, I*** bzw. I**** werden entsprechende Ausgangsmaterialien II*, II**, II*** bzw. II****; III*, III**, III*** bzw. III****; IV*, IV**, IV*** bzw. IV**** oder V*, V**, V*** bzw. V****, worin die Substituenten die entsprechende Bedeutung haben, umgesetzt.

Werden die Biphenylylaminoalkansäuren der Formel II unter Schutz der Carboxylgruppe zur Reaktion gebracht, so gelangen solche Vertreter zum Einsatz, deren Schutzgruppen nicht mit den Acylderivaten III reagieren. Geeignete Vertreter sind beispielsweise Ester von Alkanolen, u.a. mit 1 bis 5 Kohlenstoffatomen, oder Phenalkanolen, wie Methyl-, Propyl-, Butyl-, Benzyl- oder Phenethylester, gegebenenfalls auch Lösungen mit anorganischen oder organischen Basen, wie Alkali- oder Erdalkalimetallhydroxiden, Ammoniak, tertiären Stickstoffbasen (z.B. Triethylamin, Pyridin).

In den Acylderivaten III ist eine Fluchtgruppe $R^7$ beispielsweise eine Hydroxygruppe, ein Halogenatom, vorzugsweise ein Chlor- oder Bromatom, eine Alkylsulfonyloxy- oder Benzolsulfonyloxygruppe, wie eine Mesyloxy- oder p-Tolylsulfonyloxygruppe, eine Alkoxygruppe, vorzugsweise eine Methoxy- oder Ethoxygruppe, eine Alkylmercaptogruppe, wie eine Methylmercapto- oder Ethylmercaptogruppe.

Die Reaktion der Biphenylylaminoalkansäuren II mit den Acylderivaten III wird nach an sich bekannten Verfahren durchgeführt. Die Umsetzung wird in geeigneten Lösungsmitteln, wie Wasser oder Kohlenwasserstoffen, z.B. Benzol, Toluol, Xylol, oder Ethern, z.B. Tetrahydrofuran, Dioxan, 1,2-Dimethoxyethan, oder Ketonen, z.B. Ethylmethylketon, oder Amiden, z.B. Dimethylformamid, oder Sulfoxiden z.B. Dimethylsulfoxid, durchgeführt. Zweckmäßigerweise wird die Acylierung, wenn $R^7$ eine Fluchtgruppe bedeutet, in Gegenwart eines säurebindenden Mittels (Protonenacceptors) vorgenommen. Als solche sind beispielsweise geeignet Alkalimetallhydroxide, wie Natriumhydroxid, Kaliumhydroxid, oder Alkalimetallcarbonate, wie Natriumcarbonat, Kalimcarbonat, oder tertiäre Amine, wie Pyridin, Triethylamin, Ethyldiisopropylamin. Stellen die Acylderivate III Säureanhydride dar, d.h. bedeutet $R^7$ eine $R^1$—CO—O-Gruppe, so genügt auch das Erhitzen der Verbindung II und III in einem inerten Lösungsmittel.

Die Reaktion kann in weiten Grenzen variiert werden, z.B. —20 bis +100°C, wobei Temperaturen um Raumtemperatur (10 bis 30°C) bevorzugt sind. Werden die Verbindungen II unter Schutz der Carboxylgruppe acyliert, so wird nach der Acylierung die Schutzgruppe in üblicher Weise wieder abgespalten. Werden Salze als Schutzgruppe eingesetzt, so erfolgt die Freisetzung der erhaltenen Säuren I durch Umsetzung mit einer geeigneten Mineralsäure, wie Salzsäure, Schwefelsäure etc. Werden Ester als Schutzgruppen eingesetzt, so folgt auf die Acylierung die Verseifung des Reaktionsproduktes zu Verbindungen der Formel I. Die Verseifung wird vorzugsweise mit einer alkoholischen (z.B. ethanolischen) Alkalimetallhydroxid- (z.B. Kaliumhydroxid-) Lösung bei Raumtemperatur vorgenommen, gegebenenfalls unter Zusatz eines inerten Verdünnungsmittels, wie Dioxan oder Benzol.

Die Ausgangsverbindungen der Formel II werden nach verschiedenen an sich bekannten Verfahren hergestellt. So werden sie durch Umsetzung von Halogenalkansäuren der Formel VI, vorzugsweise unter Schutz der Carboxylgruppe als Estergruppe, mit einem Biphenylylamin der Formel VII

$$R^8—C_nH_{2n}—COOH \quad (VI) \qquad A—NH_2 \quad (VII),$$

worin n und A die oben angegebene Bedeutung haben und $R^8$ ein Halogenatom, vorzugsweise ein Chlor- oder Bromatom, darstellt, erhalten. Die Reaktion wird zweckmäßig in Gegenwart eines inerten Lösungsmittels, z.B. Benzol, Cyclohexan, Diethylether, unter Zusatz eines Protonenacceptors durchgeführt. Als solcher dient beispielsweise ein Überschuß des zur Reaktion eingesetzten Amins VII. Gewünschtenfalls kann jedoch auch ein anderer Protonenacceptor hinzugefügt werden.

Die Vorprodukte II werden auch durch Umsetzung eines Alkensäureesters VIII

$$CH_2{=}CH—C_{n-2}H_{2n-4}—CO—O—R^9 \quad (VIII),$$

worin n die oben angegebene Bedeutung hat und $R^9$ eine Alkylgruppe mit 1 bis 5 Kohlenstoffatomen oder eine Benzylgruppe darstellt, mit einem Biphenylylamin VII erhalten.

Die Reaktion wird zweckmäßig in Gegenwart von inerten Lösungsmitteln, wie Kohlenwasserstoffen, z.B. Benzol, Toluol, Xylol, ode Ethern, z.B. Tetrahydrofuran, Dioxan, 1,2-Dimethoxyethan, oder

Ketonen, z.B. Ethylmethylketon, oder Amiden, z.B. Dimethylformamid, oder Sulfoxiden, z.B. Dimethylsulfoxid, oder Nitrilen, z.B. Acetonitril, durchgeführt. Zweckmäßigerweise werden die Reaktionspartner und das Lösungsmittel unter Rückflußkühlung erhitzt.

Die Schutzgruppe $R^9$ wird zweckmäßigerweise erst nach Umsetzung der gewonnen Zwischenprodukte II mit den Acylderivaten III unter Erhalt der Endprodukte I abgespalten.

Die Ausgangsverbindungen II werden ferner durch Solvolyse von funktionellen Biphenylylaminoalkansäurederivaten der allgemeinen Formel IX

$$A—NH—C_nH_{2n}—G \qquad (IX),$$

worin A, n und G die oben angegebene Bedeutung haben, nach dem Fachmann bekannten Solvolyseverfahren erhalten. Zweckmäßige Ausführungsformen werden unter der Verfahrensmaßnahme c) beschrieben.

Die Vorprodukte II werden weiterhin durch Umsetzung von Biphenylylaminen VII mit Lactonen der allgemeinen Formel X

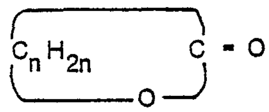

(X),

worin n die oben angegebene Bedeutung hat, in an sich bekannter Weise erhalten. Die Umsetzung erfolgt beispielsweise durch Erhitzen von VII und X in inerten Lösungsmitteln, wie Ethern, z.B. Diethylether, Tetrahydrofuran, oder Nitrilen, z.B. Acetonitril. Die erhaltene Säure kann gewünschtenfalls im Anschluß daran in die entsprechenden Ester, z.B. durch Erhitzen in den entsprechenden Alkoholen in Gegenwart einer Mineralsäure, wie Salzsäure, Schwefelsäure, übergeführt werden.

Die Zwischenprodukte II werden alternativ durch Hydrierung von Biphenylyliminoalkansäuren XI

$$A—N{=}CH—(CH_2)_{n-1}—COOH \qquad (XI),$$

worin A und n die oben angegebene Bedeutung haben, gegebenenfalls unter Schutz der Carboxylgruppe, in an sich bekannter Weise erhalten. Die Hydrierung erfolgt beispielsweise mit Raney-Nickel unter Wasserstoffdrücken von 1 bis 250 atm bei Raumtemperatur in absolutem Ethanol.

Die Säuren XI sind durch Umsetzung der Biphenylylamine VII mit Oxosäureestern XII

$$O{=}CH—(CH_2)_{n-1}—CO—O—R^9 \qquad (XII),$$

worin n und $R^9$ die oben angegebene Bedeutung haben, zugänglich.

Die Hydrierung gemäß Verfahrensvariante b) erfolgt nach Methoden, wie sie dem Fachman bekannt sind. So werden die Biphenylylaminoalkensäuren IV mit Wasserstoff in Gegenwart eines Übergangsmetall- oder Edelmetall-Katalysators oder der entsprechenden Oxide oder Komplexe in inerten Lösungsmitteln hydriert. Geeignete Metalle sind z.B. Platin, Palladium, Iridium, Rhodium. Eine Übersicht über die Hydrierungsverfahren findet sich u.a. in Kirk-Othmer *11*, 418—462; Ullmann *10*, 109—114, 541—555; *14* 630—649. Die Abspaltung einer gegebenenfalls vorhandenen Schutzgruppe erfolgt in üblicher Weise.

Die Alkensäuren IV werden beispielsweise aus den Halogenalkensäureestern XIII

$$R^{10}—C_nH_{2n-2}—CO—O—R^9 \qquad (XIII),$$

worin $R^9$ und n die oben angegebene Bedeutung haben und $R^{10}$ ein Halogenatom, vorzugsweise ein Bromatom bedeutet, durch Aminierung mit einem Biphenylylamin VII, Acylierung mit einem Acylderivat III und gegebenenfalls anschließende Verseifung gewonnen. Die Herstellung erfolgt nach an sich bekannten Verfahren, Halogenierung (Herstellung von XIII) und Aminierung z.B. analog J. Heterocycl.Chem. 8 (1971) 21; Acylierung und Verseifung werden analog der Beschreibung der vorliegenden Anmeldung durchgeführt.

Die Halogenalkansäuren VI und Biphenylylamine VII sind bekannte Verbindungen oder werden nach Analogieverfahren hergestellt; beispielsweise sind die Halogenalkansäuren VI durch Solvolyse, wie Hydrolyse oder Alkoholyse, der entsprechenden Lactone und anschließende Halogenierung zugänglich. Die Biphenylamine VII werden durch Reduktion entsprechender Nitroverbindungen, die durch Nitrierung korrespondierender Biphenyle erhältlich sind, gewonnen.

Die Solvolyse gemäß Verfahrensvariante c) erfolgt nach dem Fachmann bekannten Verfahren. Unter einem funktionellen Säurederivat wird hierbei ein Abkömmling verstanden, dessen funktionelle Gruppe G durch Solvolyse in die freie Carboxylgruppe überführbar ist. Typische Vertreter sind beispielsweise solche, worin G eine —CN-Gruppe oder eine

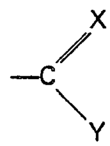 -Gruppe

bedeutet, worin

X ein Sauerstoff- oder ein Schwefelatom oder ein substituiertes Stickstoffatom, insbesondere eine Imino-, Alkylimino- oder Hydroxyiminogruppe darstellt und

Y eine Hydroxygruppe oder einen monovalenten eliminierbaren elektrophilen Rest, insbesondere eine freie oder substituierte Aminogruppe, vorzugsweise eine Monoalkyl- oder Dialkyl- oder Arylaminogruppe, eine Hydroxyamino- oder Hydrazinogruppe, eine Hydrazobenzolgruppe, eine 2-Hydroxyethylaminogruppe, eine freie oder substituierte Mercaptogruppe, vorzugsweise eine Alkylthiogruppe, eine substituierte Hydroxygruppe, vorzugsweise eine Alkoxygruppe, einen Azido-, einen Chlor- oder Bromrest, eine Morpholinogruppe oder eine Piperidinogruppe, bedeutet, wobei Y nicht eine Hydroxygruppe ist, wenn X ein Sauerstoffatom darstellt.

Unter einem Alkylrest einer Alkylimino-, einer Monoalkylamino-, einer Dialkylamino-, einer Alkylthio- und einer Alkoxygruppe wird ein Alkylrest mit bis zu 5 Kohlenstoffatomen, unter einem Arylrest einer Arylaminogruppe wird ein Arylrest mit bis zu 10 Kohlenstoffatomen verstanden.

Bevorzugte Vertreter der Säurederivate V sind solche, in denen G eine —CN-Gruppe oder eine

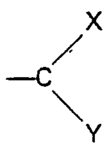 -Gruppe

darstellt, worin

X ein Sauerstoffatom, ein Schwefelatom oder eine Iminogruppe und

Y einen Amino-, Monoalkylamino-, Dialkylamino-, Phenylamino-, Alkoxy-, Alkylthio-, Chlor- oder Bromrest bedeutet.

Besonders bevorzugte Vertreter der Säurederivate V sind die entsprechenden Säureamide, Säurealkylester und Nitrile, d.h. solche Verbindungen der Formel V, in denen G eine $CO—NH_2—$, $—CO—NH—R^9—$, $—CO—NR^9_2—$, $—CO—O—R^9—$ oder —CN-Gruppe darstellt und $R^9$ die oben angegebene Bedeutung hat. Sie stellen wertvolle Zwischenprodukte für die Herstellung der Verbindungen I und ihrer Salze dar.

Zur Solvolyse der funktionellen Carbonsäurederivate V verwendet man ein wasserabgebendes Medium, das ganz oder teilweise aus Wasser bzw. aus bei den Hydrolysebedingungen wasserabspaltenden Mitteln besteht. Die Reaktion kann als Homogenreaktion geführt werden, in welchem Fall man meist in Gegenwart eines polaren organischen Lösungsmittels oder eines Lösungsvermittlers arbeitet. Vorteilhafterweise werden als Lösungsmittel beispielsweise niedermolekulare Alkohole, Dioxan, Aceton, niedermolekulare Carbonsäuren, N-Methylpyrrolidon, Sulfolan oder Dimethylsulfoxid verwendet. Man kann aber die Hydrolyse auch als Heterogenreaktion führen. Der pH-Wert des wasserabgebenden Mediums richtet sich nach der chemischen Natur des eingesetzten Säurederivates, aber auch nach der Natur der gewünschten Verbindung der allgemeinen Formel I, er kann daher neutral, sauer basisch sein. Er wird mit Säuren, Basen oder Puffern auf den gewünschten Wert eingestellt.

Die Hydrolysetemperaturen liegen zwischen 0°C und dem Siedepunkt des wasserabgebenden Mediums, im allgemeinen zwischen 0° und 150°C, insbesondere zwischen 20° und 120°C. Die Hydrolysetemperaturen hängen im einzelnen auch davon ab, ob unter Druck oder ohne Druck gearbeitet wird. Die Reaktionszeiten liegen je nach Ansatz, Reaktionstemperaturen und übrigen Reaktionsparametern zwischen 10 Minuten und 20 Stunden. Nach beendeter Hydrolyse werden die Säuren I nach üblichen Methoden, z.B. durch Umkristallisation oder durch Ansäuern ihrer Lösungen, gegebenenfalls unter Einengung ihrer Lösungen, isoliert. Zu ihrer Reingung kann deren alkalische Lösung mit einem organischen Lösungsmittel, das mit der alkalischen Lösung nicht mischbar ist, beispielsweise Diethylether, Benzol, Chlorbenzol, Chloroform oder Methylenchlorid, extrahiert werden.

Die Carbonsäurederivate V werden nach dem Fachmann geläufigen Methoden erhalten. Beispielsweise werden sie durch Reaktion von funktionellen Halogenalkansäurederivaten XIV

$$R^8—(CH_2)_n—G \qquad (XIV),$$

worin $R^8$, n und G die oben angegebene Bedeutung haben, mit Biphenylylaminen VII und anschließende Acylierung mit Acylderivaten III erhalten.

Die Überführung der Säuren der allgemeinen Formel I oder der Ausgestaltungen I*, I**, I*** bzw. I**** in ihre Salze kann durch direkte alkalische Hydrolyse der Säurederivate der allgemeinen Formel V

erfolgen. Als alkalischer Reaktionspartner wird diejenige anorganische oder organische Base verwendet, deren Salz gewünscht wird. Man erhält die Salze aber auch, indem man die Säuren der allgemeinen Formel I mit dem stöchiemetrischen Äquivalent an entsprechender Base, z.B. Natriumhydroxid oder Natriumalkoholat, umsetzt, oder leicht lösliche Salze durch doppelte Umsetzung in schwer lösliche Salze überführt, oder beliebige Salze in pharmakologisch verträgliche Salze überführt.

Die folgenden Beispiele erläutern die Erfindung näher, ohne sie einzuschränken. Die Abkürzung F. bzw. Sdp. bedeutet Schmelzpunkt bzw. Siedepunkt.

## Beispiele

### Beispiel 1

*4-[p-Chlor-N-(biphenyl-2-yl)-benzamido]-buttersäure*

$R_1$ = p-Chlorphenyl, A = Biphenyl-2-yl, n = 3

a) *4-(Biphenyl-2-yl)aminobuttersäure-ethylester*

30,0 g 2-Aminobiphenyl, 23,4 g Ethyldiisopropylamin und 35,4 g 4-Brombuttersäureethylester werden zusammen unter Rühren 3 Stunden auf 150° erhitzt. Nach dem Erkalten wird das Reaktionsprodukt mit Diethylether verrührt, das ausgeschiedene Salz abfiltriert und der nach dem Abdampfen des Ethers verbleibende Rückstand aus isopropylalkohol umkristallisiert.

Es werden 32,0 g (63,7% d.Th.) 4-(Biphenyl-2-yl)-aminobuttersäure-ethylester, F. 60—62°, erhalten.

b) *4-[p-Chlor-N-(biphenyl-2-yl)-benzamido]-buttersäure-ethylester*

9,8 g p-Chlorbenzoylchlorid werden bei Raumtemperatur zu einer Lösung von 16,0 g 4-(Biphenyl-2-yl)-aminobuttersäure-ethylester und 7,3 g Ethyldiisopropylamin in 70 ml Benzol innerhalb 30 Minuten unter Rühren zugetropft. Nach einer weiteren Stunde wird der Niederschlag abfiltriert, das Filtrat eingedampft und der Eindampfrückstand aus Cyclohexan umkristallisiert. Es werden 18,0 g (75,6% d.Th.) 4-[p-Chlor-N-(biphenyl-2-yl)-benzamido]-buttersäure- ethylester, F. 100—102°, erhalten.

c) *4-[p-Chlor-N-(biphenyl-2-yl)-benzamido]-buttersäure*

15,0 g 4-[p-Chlor-N-(biphenyl-2-yl)-benzamido]-buttersäure-ethylester werden in 100 ml Benzol gelöst und nach Zugabe einer Lösung von 2,8 g Kaliumhydroxid in 20 ml Ethanol 5 Stunden bei Raumtemperatur gerührt. Das Lösungsmittel wird dann im Vakuum abdestilliert und der Rückstand in Wasser gelöst. Die wäßrige Lösung wird mit verdünnter Salzsäure angesäuert und der sich abscheidende Niederschlag in Methylenchlorid aufgenommen. Der nach dem Trocknen und Abdestillieren des Methylenchlorids verbleibende Rückstand wird aus einem Ethanol-Wasser-Gemisch (2:1) umkristallisiert. Es werden 10,1 g (72,1% d.Th.) 4-[p-Chlor-N-(biphenyl-2-yl)-benzamido]-buttersäure, F. 135—137°, erhalten.

### Beispiel 2

*4-[m-Trifluormethyl-N-(biphenyl-2-yl)-benzamido]-buttersäure*

$R_1$ = $\alpha,\alpha,\alpha$-Trifluor-m-tolyl, A = Biphenyl-2-yl, n = 3

a) *4-[m-Trifluormethyl-N-(biphenyl-2-yl)-benzamido]-buttersäure-ethylester*

Analog Beispiel 1b) werden 11,8 g 4-(Biphenyl-2-yl)-aminobuttersäure-ethylester und 5,4 g Ethyldiisopropylamin in 70 ml Benzol mit 8,7 g m-Trifluormethyl-benzoylchlorid umgesetzt. Das Reaktionsprodukt wird aus einer Mischung Isopropylalkohol-Wasser (1:1) umkristallisiert. Es werden 16,0 g (84,4% d.Th.) 4-[m-Trifluormethyl-N-(biphenyl-2-yl)-benzamido]-buttersäure-ethylester, F. 65—67°, erhalten.

b) *4-[-m-Trifluormethyl-N-(biphenyl-2-yl)-benzamido]-buttersäure*

16,0 g 4-[m-Trifluormethyl-N-(biphenyl-2-yl)-benzamido]-buttersäure-ethylester in 50 ml Benzol werden mit einer Lösung von 3,4 g Kaliumhydroxid in 25 ml Ethanol versetzt und 8 Stunden bei Raumtemperatur gerührt. Bei der Aufarbeitung analog Beispiel 1c) werden 13,7 g (91,3% d.Th.) 4-[m-Trifluormethyl-N-(biphenyl-2-yl)-benzamido]-buttersäure, F. 156—157°C, erhalten.

### Beispiel 3

*4-[p-Fluor-N-(biphenyl-2-yl)-benzamido]-buttersäure*

$R_1$ = p-Fluorphenyl, A = Biphenyl-2-yl, n = 3

a) *4-[p-Fluor-N-(biphenyl-2-yl)-benzamido]-buttersäure-ethylester*

Analog Beispiel 1b) werden 10,0 g 4-(Biphenyl-2-yl)-aminobuttersäure-ethylester und 4,6 g Ethyldiisopropylamin in 50 ml Benzol mit 5,6 g p-Fluorbenzoylchlorid umgesetzt. Das Reaktionsprodukt wird aus Cyclohexan umkristallisiert. Es werden 12,1 g (84,6% d.Th.) 4-[p-Fluor-N-(biphenyl-2-yl)-benzamido]-buttersäure-ethylester, F. 83—84°, erhalten.

b) *4-[p-Fluor-N-(biphenyl-2-yl)-benzamido]-buttersäure*

11,2 g 4-[p-Fluor-N-(biphenyl-2-yl)-benzamido]-buttersäure-ethylester in 50 ml Benzol werdet mit einer Lösung von 2,2 g Kaliumhydroxid in 20 ml Ethanol versetzt und 8 Stunden bei Raumtemperatur gerührt. Bei der Aufarbeitung analog Beispiel 1c) werden 7,1 g (71,7% d.Th.) 4-[p-Fluor-N-(biphenyl-2-yl)-benzamido]-buttersäure, F. 120—122°, erhalten.

Beispiel 4

*4-[5-Chlor-2-methoxy-N-(biphenyl-2-yl)-benzamido]-buttersäure*

$R_1$ = 5-Chlor-2-methoxyphenyl, A = Biphenyl-2-yl, n = 3

Analog Beispiel 1 werden 10,0 g 4-(Biphenyl-2-yl)-amino-buttersäure-ethylester und 4,6 g Ethyldiisopropylamin in 50 ml Benzol mit einer Lösung von 7,3 g 5-Chlor-2-methoxybenzoylchlorid in 20 ml Benzol umgesetzt. Als Reaktionsprodukt werden 15,0 g (94,1% d.Th.) 4-[5-Chlor-2-methoxy-N-(biphenyl-2-yl)-benzamido]-buttersäure-ethylester als zähes, nicht destillierbares Öl erhalten. Die Verseifung dieses Esters ergibt 12,4 g (88% d.Th.) 4-[5-Chlor-2-methoxy-N-(biphenyl-2-yl)-benzamido]-buttersäure, F. 160—162°.

Beispiel 5

*4-[N-(Biphenyl-2-yl)-benzamido]-buttersäure*

$R_1 = CH_3—$, A = Biphenyl-2-yl, n = 3

Analog Beispiel 1 werden 16,0 g 4-(Biphenyl-2-yl)-aminobuttersäure-ethylester und 7,3 g Ethyldiisopropylamin in 70 ml Benzol mit 4,4 g Acetylchlorid umgesetzt. Als Reaktionsprodukt werden 14,0 g (76,2% d.Th.) 4-[N-(Biphenyl-2-yl)-acetamido]-buttersäure-ethylester als zähes, nicht destillierbares Öl erhalten. Die Verseifung dieses Esters ergibt nach dem Umkristallisieren des erhaltenen Rohprodukts aus Isopropylalkohol 10,1 g (79,0% d.Th.) 4-[N-(Biphenyl-2-yl)-acetamido]-buttersäure, F. 124—125°. 124—125°.

Beispiel 6

*4-[N-(Biphenyl-2-yl)-crotonoylamido]-buttersäure*

$R_1 = CH_3—CH=CH—$, A = Biphenyl-2-yl, n = 3

Analog Beispiel 1 werden 10,0 g 4-(Biphenyl-2-yl)-amino-buttersäure-ethylester und 4,6 g Ethyldiisopropylamin in 70 ml Benzol mit 3,8 Crotonoylchlorid umgesetzt. Als Reaktionsprodukt werden 10,1 g (81,5% d.Th.) 4-[N-(Biphenyl-2-yl)-crotonoylamido]-buttersäure-ethylester als zähes, nicht destillierbares Öl erhalten. Die Verseifung dieses Esters ergibt nach dem Umkristallisieren des erhaltenen Rohprodukts aus Isopropylalkohol 5,1 g (55% d.Th.) 4-[N-(Biphenyl-2-yl)-crotonoylamido]-buttersäure, F. 127—128°.

Beispiel 7

*5-[p-Chlor-N-(biphenyl-2-yl)-benzamido]-valeriansäure*

$R_1$ = p-Chlorphenyl, A = Biphenyl-2-yl, n = 4

50,0 g 2-Aminobiphenyl, 38,2 g Ethyldiisopropylamin und 61,9 g 5-Bromvaleriansäure-ethylester werden analog Beispiel 1a) umgesetzt. Es werden 80,0 g 5-(Biphenyl-2-yl)-aminovaleriansäure-ethylester als nicht kristallisierendes Öl erhalten.

30,0 g dieses Esters werden analog Beispiel 1b) mit 13,1 g Ethyldiisopropylamin und 17,1 g p-Chlorbenzoylchlorid in 70 ml Benzol umgesetzt und der als öliges Zwischenprodukt erhaltene 5-[p-Chlor-N-(biphenyl-2-yl)-benzamido]-valeriansäurethylester analog Beispiel 1c) verseift. Es werden 22,1 g (53,7% d.Th) 5-[p-Chlor-N-(biphenyl-2-yl)-benzamido]-valeriansäure, F. 170—173°, erhalten.

Beispiel 8

*5-[o-Hydroxy-N-(biphenyl-2-yl)-benzamido]-valeriansäure*

$R_1$ = o-Hydroxyphenyl, A = Biphenyl-2-yl, n = 4

15,0 g 5-(Biphenyl-2-yl)-aminovaleriansäure-ethylester werden analog Beispiel 1b) mit 6,6 g Ethyldiisopropylamin und 10,0 g o-Acetoxybenzoylchlorid in 70 ml Benzol umgesetzt. Als Reaktionsprodukt werden 17,0 g (73,4% d.Th.) 5-[o-Acetoxy-N-(biphenyl-2-yl)-benzamido]-valeriansäure-ethylester als zähes, nicht destillierbares Öl erhalten. Die Verseifung dieses Esters ergibt 9,1 g (63,2% d.Th.) 5-[o-Hydroxy-N-(biphenyl-2-yl)-benzamido]-valeriansäure, F. 138—139°C.

Beispiel 9

*6-[p-Chlor-N-(biphenyl-2-yl)-benzamido]-capronsäure*

$R_1$ = p-Chlorphenyl, A = Biphenyl-2-yl, n = 5

20,0 g 2-Aminobiphenyl, 15,3 g Ethyldiisopropylamin und 26,3 g 6-Bromcapronsäure-ethylester werden analog Beispiel 1a) umgesetzt. Es werden 36,0 g 6-(Biphenyl-2-yl)-aminocapronsäure-ethylester als nicht kristallisierendes Öl erhalten. 18,0 g dieses Esters werden analog Beispiel 1b) mit 7,5 g Ethyldiisopropylamin und 10,1 g p-Chlorbenzoylchlorid in 70 ml Benzol umgesetzt. Das Reaktionsprodukt wird über eine Kieselgelsäule (Laufmittel Methylenchlorid) chromatographisch gereinigt. Es werden 11,5 g (44,2% d.Th.) 6-[p-Chlor-N-(biphenyl-2-yl)-benzamido]-capronsäure-ethylester als zähes, nicht destillierbares Öl erhalten. Die Verseifung dieses Esters ergibt 8,1 g (75,1% d.Th) 6-[p-Chlor-N-(biphenyl-2-yl)-benzamido]-capronsäure, F. 93—95°.

Beispiel 10

*6-[5-Chlor-2-methoxy-N-(biphenyl-2-yl)-benzamido]-capronsäure*

$R_1$ = 5-Chlor-2-methoxyphenyl, A = Biphenyl-2-yl, n = 5

80, g 6-(Biphenyl-2-yl)-aminocapronsäure-ethylester und 3,3 g Ethyldiisopropylamin in 50 ml Benzol werden mit einer Lösung von 5,3 g 5-Chlor-2-methoxybenzoylchlorid in 20 ml Benzol analog Beispiel 1a) umgesetzt. Als Reaktionsprodukt werden 11,1 g (90% d.Th.) 6-[5-Chlor-2-methoxy-N-(biphenyl-2-yl)-benzamido]-capronsäure-ethylester als zähes, nicht destillierbares Öl erhalten. Die Verseifung dieses Esters ergibt 7,0 g (67% d.Th.) 6-[5-Chlor-2-methoxy-N-(biphenyl-2-yl)-benzamido]-capronsäure, F. 164—165°.

Beispiel 11

*6-[N-(Biphenyl-2-yl)-isobutyramido]-capronsäure*

$R_1$ = —$CH(CH_3)_2$, A = Biphenyl-2-yl, n = 5

Analog Beispiel 10 werden aus 10,0 g 6-(Biphenyl-2-yl)-aminocapronsäure-ethylester, 4,2 g Ethyldiisopropylamin und 3,4 Isobutyrylchlorid in 70 ml Benzol 8,9 g (72,6% d.Th.) 6-[N-(Biphenyl-2-yl)-isobutyramido]-capronsäureethylester als zähes, nicht destillierbares Öl erhalten. Die Verseifung dieses Esters ergibt 6,1 g (74% d.Th.) 6-[N-(Biphenyl-2-yl)-isobutryamido]-capronsäure, F. 120—121°.

Beispiel 12

*4-[p-Chlor-N-(biphenyl-4-yl)-benzamido]-buttersäure*

$R_1$ = p-Chlorphenyl, A = Biphenyl-4-yl, n = 3

a) *4-(Biphenyl-4-yl)-aminobuttersäure-ethylester*

Aus 25,0 g 4-Aminobiphenyl, 19,2 g Ethyldiisopropylamin und 28,9 g 4-Brombuttersäure-ethylester werden analog Beispiel 1a) erhalten: 24,0 g (57,3% d.Th.) 4-(Biphenyl-4-yl)-aminobuttersäure-ethylester, F. 82—84°.

b) *4-[p-Chlor-N-(biphenyl-4-yl)-benzamido]-buttersäure*

Analog Beispiel 1b) werden 10,0 g 4-(Biphenyl-4-yl)-aminobuttersäure-ethylester und 4,6 g Ethyldiisopropylamin in 70 ml Benzol mit 6,2 g p-Chlorbenzoylchlorid umgesetzt. Es werden 9,4 g (62,9% d.Th.) 4-[p-Chlor-N-(biphenyl-4-yl)-benzamido]-buttersäure-ethylester als zähes, nicht destillierbares Öl erhalten. Die Verseifung dieses Esters analog Beispiel 1c) ergibt 7,0 g (79,7% d.Th.) 4-[p-Chlor-N-(biphenyl-4-yl)-benzamido]-buttersäure, F. 192—194°.

Beispiel 13

*4-[p-Chlor-N-(6-methoxy-biphenyl-3-yl)-benzamido]-buttersäure*

$R^1$ = p-Chlorphenyl, A = 6-Methoxy-biphenyl-3-yl, n = 3

a) *4-(6-Methoxy-biphenyl-3-yl)-aminobuttersäure-ethylester*

29,5 g 5-Amino-2-methoxy-biphenyl, 19,2 g Ethyldiisopropylamin und 28,9 g 4-Brombuttersäure-ethylester werden zusammen unter Rühren 4 Stunden auf 125° erhitzt. Nach dem Erkalten wird das Reaktionsprodukt mit Diethylether verrührt, vom ausgeschiedenen Salz abfiltriert und der nach dem Abdampfen des Ethers verbleibende Rückstand aus Isopropylalkohol umkristallisiert. Es werden 22,5 g (48,5% d.Th.) 4-(6-Methoxy-biphenyl-3-yl)-aminobuttersäure-ethylester, F. 71—72°, erhalten.

b) *4-[p-Chlor-N-(6-methoxy-biphenyl-3-yl)-benzamido]-buttersäure*

8,0 g 4-(6-Methoxy-biphenyl-3-yl)-aminobuttersäure-ethylester, 3,3 g Ethyldiisopropylamin in 50 ml Benzol werden mit 4,6 g p-Chlorbenzoylchlorid analog Beispiel 1b) umgesetzt. Das Reaktionsprodukt wird in 50 ml Benzol gelöst und nach Zugabe einer Lösung von 2,3 g Kaliumhydroxid in 20 ml Ethanol 8 Stunden bei Raumtemperatur gerührt. Nach dem Abdestillieren des Lösungsmittels wird der Rückstand in Wasser gelöst, die Lösung mit verdünnter Salzsäure angesäuert und mit Diethylether extrahiert. Der nach dem Abdampfen des Ethers verbleibende Rückstand wird aus Essigester-Petrolether (1:1) umkristallisiert. Es werden 8,1 g (73,5% d.Th.) 4-[p-Chlor-N-(6-methoxybiphenyl-3-yl)-benzamido]-buttersäure, F. 114—116°, erhalten.

*Beispiel 14*

*4-[5-Chlor-2-methoxy-N-(6-methoxy-biphenyl-3-yl)-benzamido]-buttersäure*

$R^1$ = 5-Chlor-2-methoxyphenyl, A = 6-Methoxy-biphenyl-3-yl, n = 3

Analog Beispiel 13 werden durch Umsetzung von 7,0 g 4-(6-Methoxy-biphenyl-3-yl)-aminobuttersäure-ethylester mit 4,6 g 5-Chlor-2-methoxy-benzoylchlorid und anschließender Verseifung des Zwischenprodukts 7,1 g (70,1% d.Th.) 4-[5-Chlor-2-methoxy-N-(6-methoxy-biphenyl-3-yl)-benzamido]-buttersäure, F. 155—156°, erhalten.

Beispiel 15

*6-[2,4-Dichlor-N-(biphenyl-2-yl)-benzamido]-capronsäure*

$R^1$ = 2,4-Dichlorphenyl, A = Biphenyl-2-yl, n = 5

Nach dem in Beispiel 9 angegebenen Verfahren, wobei jedoch anstelle von p-Chlorbenzoylchlorid 2,4-Dichlorbenzoylchlorid verwendet wird, erhält man 6-[2,4-Dichlor-N-(biphenyl-2-yl)-benzamido]-capronsäure, F. 112—113° (aus Essigester-Petrolether 1:1).

Beispiel 16

*4-[p-Chlor-N-(1',2',3',4',5',6'-hexahydrobiphenyl-4-yl)-benzamido]-buttersäure*

$R^1$ = p-Chlorphenyl, A = 1',2',3',4',5',6'-Hexahydrobiphenyl-4-yl, n = 3

Nach dem in Beispiel 1a—c angegebenen Verfahren, wobei jedoch anstelle von 2-Aminobiphenyl p-Cyclohexylanilin eingesetzt wird, erhält man 4-[p-Chlor-N-(1',2',3',4',5',6',-hexahydrobiphenyl-4-yl)-benzamido]-buttersäure, F. 75—77° (aus Isopropylalkohol-Wasser 1:1).

Beispiel 17

*5-[p-Chlor-N-(biphenyl-2-yl)-benzamido]-valeriansäure*

a) *5-[N-(biphenyl-2-yl)-amino]-valeriansäure*

Eine Lösung von 8,0 g 5-(Biphenyl-2-yl)-aminovaleriansäure-ethylester (siehe Beispiel 7) in 60 ml Benzol wird mit einer Lösung von 2,2 g Kaliumhydroxid in 20 ml Ethanol vermischt. Nach 24-stündigem Stehen bei Raumtemperatur wird das Lösungsmittelgemisch im Vakuum abdestilliert, der Rückstand in Wasser gelöst, die wässerige Lösung mit Diethylether gewaschen und dann mit verdünnter Salzsäure angesäuert. Die zunächst ölige Fällung wird in Dichlormethan aufgenommen, das Lösungsmittel abdestilliert und der Rückstand aus Essigester-Petrolether 1:1 umkristallisiert. Es werden so 5,6 g (77,3% d.Th.) 5-[N-(Biphenyl-2-yl)-amino]-valeriansäure, F. 73—75°, erhalten.

b) *5-[p-Chlor-N-(biphenyl-2-yl)-benzamido]-valeriansäure*

5,4 g 5-[N-(Biphenyl-2-yl)-amino]-valeriansäure werden in 40 ml 0,2 n Natronlauge gelöst. In die klare Lösung werden unter kräftigem Rühren und laufender pH-Kontrolle 3,5 g p-Chlorbenzoylchlorid eingetropft unter gleichzeitiger Zugabe von verdünnter Natronlauge, um den pH-Wert zwischen 7 und 8 zu halten. Nach Beendigung der Säurechloridzugabe wird die Lösung noch weitere 30 Minuten bei pH 8 gerührt und dann mit verdünnter Salzsäure bis pH 3 angesäuert. Der zunächst ölig ausfallende Niederschlag kristallisiert nach einiger Zeit. Er wird abfiltriert und aus Isopropylalkohol umkristallisiert. Es werden so 7,6 g (93% d.Th.) 5-[p-Chlor-N-(biphenyl-2-yl)-benzamido]-valeriansäure, F. 170—173°, erhalten, die mit der nach Beispiel 7 erhaltenen Verbindung identisch ist.

Beispiel 18

Ampullen mit 600 mg 4-[p-Chlor-N-(biphenyl-2-yl)-benzamido]-buttersäure, Chargengröße 250 kg

25,0 kg 1,2-Propylenglykol und 150,0 kg aqua bidestillata werden vorgelegt, 15,000 kg 4-[p-Chlor-N-(biphenyl-2-yl)benzamido]buttersäure, zugegeben und anschließend wird langsam, unter Rühren, Natronlauge (10 Gew.-% NaOH) zugegeben. Wenn sich alles gelöst hat, wird der pH-Wert auf 7,5—8,0 eingestellt. 0,0625 kg Natriumpyrosulfit werden zugegeben und die Mischung wird gerührt, bis sich alles gelöst hat. Mit aqua bidestillata wird auf 250 kg aufgefüllt. Die Lösung wird in 10 ml-Ampullen abgefüllt und bei 120° 30 Minuten im Autoklaven sterilisiert.

Beispiel 19

Ampullen mit 600 mg 4-[N-(Biphenyl-2-yl)-crotonoylamido]buttersäure, Chargengröße 250 kg

50,000 kg 1,2-Propylenglykol und £50 kg aqua bidestillata werden vorgelegt. Dann werden unter Rühren 15 kg 4-[N-(Biphenyl-2-yl)-crotonoylamido]-buttersäure zugegeben. An schließend wird Natronlauge (10 Gew.-% NaOH) hinzugefügt und die Mischung dabei auf einen pH-Wert von 8,0 eingestellt. Mit aqua bidestillata wird auf 250 kg ergänzt. Die Lösung wird in 10 ml-Ampullen abgefüllt und 30 Minuten bei 120° im Autoklaven sterilisiert.

Beispiel 20

Tabletten mit 50 mg 4-[p-Fluor-N-(biphenyl-2-yl)-benzamido]buttersäure

25,000 kg 4-[p-Fluor-N-(biphenyl-2-yl)-benzamido]-buttersäure, 35,000 kg Milchzucker und 26,000 kg Maisstärke werden mit 21,500 kg Polyvinylpyrrolidon (MG~25.000) in ungefähr 6 Liter Wasser granuliert. Das Granulat wird durch ein Sieb von 1,25 mm Maschenweite gesiebt und nach dem Trocknen werden 8,000 kg Carboxymethylcellulose, 2,500 kg Talkum und 1,000 kg Magnesiumstearat zugegeben. Man verpreßt das trockene Granulat zu Tabletten von 8 mm Durchmesser, 250 mg Gewicht und einer Härte von 5—6 kg

In ähnlicher Weise werden Tabletten mit 4-[p-Chlor-N-(biphenyl-2-yl)-benzamido]-buttersäure bzw. 4-[N-(Biphenyl-2-yl)-crotonoylamido]-buttersäure hergestellt.

Beispiel 21

Tabletten mit 100 mg 4-[N-(Biphenyl-2-yl)-acetamido]-buttersäure

40,000 kg 4-[N-(Biphenyl-2-yl)-acetamido]-buttersäure, 24,000 kg Milchzucker und 16,000 kg Maisstärke werden mit 4,000 kg Polyvinylpyrrolidon (MG~25.000) in ungefähr 5,5 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einer Exzentermaschine wird das Granulat zu Tabletten von 9 mm Durchmesser, 250 mg Gewicht und einer Härte von 4—5 kg verpreßt.

MG = Molekulargewicht

Beispiel 22

Tabletten mit 300 mg 4-[p-Chlor-N-(biphenyl-2-yl)-benzamido]-buttersäure

60,000 kg 4-[p-Chlor-N-(biphenyl-2-yl)-benzamido]-buttersäure, 12,000 kg Milchzucker und 8,000 kg Maisstärke werden mit 4,000 kg Polyvinylpyrrolidon (MG~25.000) in ungefähr 6 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einem Rundläufer wird das Granulat zu Tabletten von 11 mm Durchmesser, 500 mg Gewicht und einer Härte von 6—7 kg verpreßt.

Analog werden Tabletten, die 300 mg 4-[p-Chlor-N-(biphenyl-2-yl)-benzamido]-capronsäure enthalten, hergestellt.

Beispiel 23

10.000 Kapseln mit einem Wirkstoffgehalt von 50 mg werden aus folgenden Bestandteilen hergestellt: 500 g 4-[N-(Biphenyl-2-yl)-crotonoylamido]-buttersäure, 495 g mikrokristalline Cellulose und 5 g amorphe Kieselsäure. Der Wirkstoff in feingepulverter Form, die Cellulose und die Kieselsäure werden gut gemischt und in Hartgelatinekapseln Größe 4 abgefüllt.

Pharmakologie

Die Acylbiphenylylaminosäuren üben eine starke Schutzwirkung auf Magen und Leber von Ratten aus, daneben steigern sie die Pankreas- und Gallesekretion von Ratten, wobei sie sich bekannten Handelspräparaten, z.B. Piprozolin, Carbenoxolon, überlegen erweisen. Außerdem bewirken sie eine Hemmung der Glukosebildung aus Lactat und Pyruvat in der Leber von Ratten, wobei sie sich bekannten Handelspräparaten, z.B. Buformin, Phenformin, überlegen erweisen.

In den anschließenden Tabellen werden die untersuchten Verbindungen durch eine laufende Nummer gekennzeichnet, die wie folgt zugeordnet ist:

| Lfd. Nr. | Name der Verbindung |
|---|---|
| 1 | Piprozolin |
| 2 | Carbenoxolon |
| 3 | Buformin |
| 4 | Phenformin |
| 5 | 4-[N-(Biphenyl-2-yl)- acetamido]-buttersäure |
| 6 | 4-[p-Chlor-N-(biphenyl-4-yl)-benzamido]-buttersäure |
| 7 | 6-[p-Chlor-N-(biphenyl-2-yl)-benzamido]-capronsäure |
| 8 | 4-[m-Trifluormethyl-N-(biphenyl-2-yl)-benzamido]-buttersäure |
| 9 | 4-[p-Fluor-N-(biphenyl-2-yl)-benzamido]-buttersäure |
| 10 | 4-[N-(Biphenyl-2-yl)-crotonoylamido] buttersäure |
| 11 | 5-[p-Chlor-N-(biphenyl-2-yl)-benzamido]-valeriansäure |
| 12 | 6-[2,4-Dichlor-N-(biphenyl-2-yl)-benz-amido]capronsäure |
| 13 | 6-[5-Chlor-2-methoxy-N-(biphenyl-2-yl)-benzamido]-capronsäure |
| 14 | 4-[p-Chlor-N-(biphenyl-2-yl)-benzamido]-buttersäure |
| 15 | 5-[o-Hydroxy-N-(biphenyl-2-yl)-benzamido]-valeriansäure |
| 16 | 4-[2-Methoxy-5-chlor-N-(biphenyl-2-yl)-benz-amido]-buttersäure |
| 17 | 6-[-N-((biphenyl-2-yl)-isobutyramido]-capron-säure |
| 18 | 4-[p-Chlor-N-(6-methoxy-biphenyl-3-yl)-benzamido]-buttersäure |
| 19 | 4-[2-Methoxy-5-chlor-N-(6-methoxy-biphenyl-3-yl)-benzamido]-buttersäure |
| 20 | 4-[p-Chlor-N-(1′,2′,3′,4′,5′,6′-hexahydro biphenyl-4-yl)-benzamido]-buttersäure |

Tabelle I gibt die Magenschutzwirkung (Rückbildung der durch Pylorusligatur und Verabreichung von 100 mg/kg p.o. Acetylsalicylsäure provozierten Magenulcera) nach intraduodenaler Applikation bei der Ratte, die letale Wirkung nach intraperitonealer Gabe bei der Maus sowie den therapeutischen Quotienten ($TQ = LD_{50}/ED_{50}$) von Vertretern der erfindungsgemäßen Verbindungen wieder.

TABELLE I

Magenschutzwirkung

| lfd. Nr. | Toxizität $LD_{50}$ [mg /kg] (Maus, i.p.) | Antiulcerogene Wirkung /Ratte $ED_{50}$*) [mg /kg, i.d.] | TQ $LD_{50}/ED_{50}$ |
|---|---|---|---|
| 2 | 120 | 50 | 2.4 |
| 5 | 750 | 55 | 13.6 |
| 6 | 120 | 14 | 8.6 |
| 7 | 130 | 22 | 5.9 |
| 8 | 110 | 10 | 11.0 |
| 9 | 210 | 40 | 5.3 |
| 10 | 300 | ~ 40 | ~ 7.5 |
| 11 | 140 | 30 | 4.7 |
| 13 | >400 **) | 10 | >40 |
| 14 | 180 | 20 | 9.0 |
| 15 | >400 | <10 | >40 |
| 18 | 120 | <10 | >12 |
| 19 | 190 | 21 | 9.0 |

*) Dosis, die den mittleren Ulcus index um 50% mindert.

**) p.o., da i.p. nicht applizierbar.

In Tabelle II werden Untersuchungen zur antihepatotoxischen Wirkung ($ED_{25:50}$) der erfindungsgemäßen Verbindungen nach oraler Applikation an wachen Ratten und die letale Wirkung nach intraperitonealer Applikation an der Maus ($LD_{50}$) sowie die therapeutischen Quotienten ($TQ=LD_{50}/ED_{50}$ bzw. $LD_{50}/ED_{25}$) wiedergegeben.

TABELLE II

Antihepatotoxischer Effekt, Toxizität und therapeutischer Quotient

| lfd. Nr. | Toxizität $LD_{50}$ [mg/kg] Maus ip. | Antihepatotoxischer Effekt $ED_{25}$*) mg/kg po. Ratte | $ED_{50}$*) | TQ $LD_{50}/ED_{25}$ | $LD_{50}/ED_{50}$ |
|---|---|---|---|---|---|
| 1 | 1070**) | 200 | >300 | 5.4 | >3.6 |
| 5 | 750 | <3 | 15 | >250 | 50 |
| 6 | 120 | 15 | 22 | 8 | 5.5 |
| 7 | 130 | ~9 | 14 | ~14.4 | 9.3 |
| 8 | 110 | 10 | 30 | 11 | 3.7 |
| 9 | 210 | 1.6 | 2.8 | 131.3 | 75 |
| 10 | 300 | 1.6 | 2.8 | 187.5 | 107.1 |
| 11 | 140 | 3 | 12 | 46.7 | 11.7 |
| 13 | >400***) | 10 | | >40 | |
| 14 | 180 | <1 | 3.5 | >180 | 51.4 |
| 17 | 260 | <30 | | >8.7 | |
| 20 | ~150 | $\lesssim$10 | | $\gtrsim$15 | |

*) $ED_{25}$ bzw. $ED_{50}$ - Dosis, die für $CCl_4$-lebergeschädigte Ratten die Hexobarbital-Narkose um 25 bzw. 50 % verkurzt.

**) $LD_{50}$ [p.o.], zit. aus Herrmann et al. Arzneim.-Forsch. 27 (1977)467.

***) p.o., da ip. nicht applizierbar.

In Tabelle III ist für Vertreter der erfindungsgemäßen Verbindungen der Einfluß auf die Gallesekretion (Cholerese) von narkotisierten Ratten nach intraduodenaler Applikation ($ED_{50}$) und die letale Wirkung an der Maus ($LD_{50}$) nach intraperitonealer Applikation sowie der therapeutische Quotient ($TQ = LD_{50}/ED_{50}$) wiedergegeben.

TABELLE III

Gallesekretion, Toxizität und therapeutischer Quotient

| lfd. Nr. | Toxizität $LD_{50}$ [mg/kg] (Maus, i.p.) | Gallesekretion $ED_{50}$***) [mg/kg] (Ratte i.d.) | TQ [$LD_{50}/ED_{50}$] |
|---|---|---|---|
| 1 | 1070** | 40 | 26.8 |
| 5 | 750 | ~15 | ~ 50 |
| 10 | 300 | – | ~ 30 |
| 19 | 190 | 1 | 190 |

**) $LD_{50}$ [p.o.], zit aus Herrmann et al. Arzneim.-Forsch. 27 (1977) 467.

***) $ED_{50}$ = Dosis, die eine Steigerung der Gallesekretion (Flüssigkeitsvolumen; 30-min-Fraktion) um maximal 50 % bewirkt.

In Tabelle IV ist für Vertreter der erfindungsgemäßen Verbindungen der Einfluß auf die Pankreassekretion von narkotisierten Ratten nach intraduodenaler Applikation ($ED_{50}$), und die letale Wirkung an der Mause ($LD_{50}$) nach intraperitonealer Applikation sowie der therapeutische Quotient ($TQ = LD_{50}/ED_{50}$) wiedergegeben.

TABELLE IV

Pankreassekretion, Toxizität und therapeutischer Quotient.

| 1fd. Nr. | Toxizität $LD_{50}$ [mg /kg] (Maus, i.p.) | Pankreassekretion $ED_{50}$*) [mg /kg] (Ratte, i.d.) | TQ [$LD_{50}$/$ED_{50}$] |
|---|---|---|---|
| 1 | 1070 **) | 35 | 31 |
| 5 | 750 | ~2 | ~ 375 |
| 6 | 120 | 1 | 120 |
| 8 | 110 | 1 | 110 |
| 9 | 210 | 2.5 | 84 |
| 12 | ~ 250 | 1.7 | ~147 |
| 13 | >400 ***) | <1.0 | >400 |
| 14 | 180 | 1 | 180 |
| 16 | 170 | 2 | 85 |
| 18 | 120 | 1.5 | 80 |
| 19 | 190 | 5 | 38 |
| 20 | ~ 150 | 1.5 | ~ 100 |

*) $ED_{50}$ = Dosis, die eine Steigerung der Pankreassekretion (Flussigkeitsvolumen; 30-min.-Fraktion) um maximal 50 % bewirkt.

**) $LD_{50}$ [p.o.], zit.aus Herrmann et al. Arzneim.-Forsch. 27 (1977) 467.

***) p.o., da ip. nicht applizierbar.

In Tabelle V werden die Ergebnisse der Untersuchung des Einflusses von Vertretern der erfindungsgemäßen Verbindungen auf die Glucosebildung aus Lactat und Pyruvat an der isoliert perfundierten Leber nüchterner Ratten, wobei die Hemmung der Glucosebildung bei einer Substanzkonzentration von 0,2 mmol/l im Perfusat und die $ED_{50}$—ermittelt aus 4 Konzentrationen im Bereich von 0.02—1.00 mmol/l—dargestellt sind, und die letale Wirkung an der Maus ($LD_{50}$) nach intraperitonealer Applikation wiedergegeben.

TABELLE V

Hemmung der Glucosebildung aus Lactat und Pyruvat in der isoliert perfundierten Rattenleber und Toxizität an der Maus

| lfd. Nr. | Glucosebildung %-Veränderung *) | $ED_{50}$ **) [mg /l] | $LD_{50}$ (i p.) [mg /kg] |
|---|---|---|---|
| 3 | −1 | >1000 | 213***) |
| 4 | −3 | >1000 | 150****) |
| 14 | −79 | 43 | 180 |
| 7 | −99 | 23 | 130 |
| 8 | −77 | 34 | 110 |
| 9 | −51 | 76 | 210 |
| 11 | −89 | 25 | 140 |
| 12 | −93 | 23 | ~ 250 |

*) Substanzkonzentration 0,2 mmol /l im Perfusat.

**) Dosis, die eine Hemmung der Glucosebildung um 50% bewirkt.

***) zitiert aus Söling, H.D., Creutzfeldt, W., Int. Biguanid-Symp., Aachen 1960, Stuttgart, Thieme Verlag.

****) zitiert nach Bertarelli, P. Boll.chim.farm. 97 (1958) 396.

Die erfindungsgemäßen Verbindungen zeichnen sich gegenüber den Vergleichsverbindungen 3 und 4 durch eine wesentlich stärkere Hemmung der Glucosebildung aus Lactat und Pyruvat aus. Während 3 und 4 bei den verwendeten Konzentrationen nahezu keine Hemmung bewirken, lassen sich mit den erfindungsgemäßen Verbindungen Hemmeffekte bis zu 99% erzielen.

Die Ermittlung der pharmakologischen Eigenschaften erfolgte nach folgenden Methoden:

*Einfluß auf die Pankreas- und Gallesekretion der narkotisierten Ratte*
*Versuchsdurchführung:*

Männliche Spraque-Dawley-Ratten (250—300 g Körpergewicht) werden mit 1,2 g/kg Urethan i.m. narkotisiert. Dann wird die Bauchhöhle medial geöffnet, der Ductus choledochus kurz oberhalb seiner Mündung in das Duodenum sowie nahe der Leberpforte ligiert, beide Gangabschnitte werden leberwärts katheterisiert.

Da bei der Ratte alle Pankreaskanäle in den mittleren Abschnitt des Ductus choledochus münden, lassen sich auf diese Weise aus dem distalen (ligierten) Gangabschnitt das Pankreassekret und aus dem proximalen Teil des Ductus choledochus die Galle getrennt ableiten.

Die sezernierten Mengen an Pankreas- und Gallensaft werden in 30 min. Abständen während 2 Stunden vor bis 3 Stunden nach der intraduodenalen Verabreichung der zu prüfenden Verbindungen (verabreichtes Flüssigkeitsvolumen 5 ml/kg) gemessen.

Die Körpertemperatur der Tiere wird mittels Heizkissen und Bestrahlung auf 36—38°C gehalten; die Temperaturkontrolle erfolgt rectal.

*Auswertung:*

Die Flüssigkeitsvolumina der 30-min.-Fraktionen nach der Substanzgabe werden jeweils auf die vor der Substanzapplikation ausgeschiedene Galle- bzw. Pankreassaftmenge (= 100%, Mittelwert aus den beiden letzten Messungen) bezogen. Die maximale prozentuale Steigerung der Pankreas- bzw.

Gallesekretion wird Abhängigkeit von der Dosis dargestellt und daraus die $ED_{50}$ durch Interpolation ermittelt.

*Prüfung auf antihepatotoxische Wirkung*
*Einfluß auf die Hexobarbitalschlafdauer der Ratte nach $CCl_4$-Leberschädigung*
*Versuchsdurchführung:*

In Anlehnung an VOGEL et al. [Arneim. Forsch. 25(1975)82] wird bei nüchternen weiblichen Sprague-Dawley-Ratten (190 ± 10 g Körpergewicht, 10 Tiere/Dosis pro Versuchsansatz) durch orale Verabreichung von Tetrachlorkohlenstoff (0,15 ml/kg $CCl_4$ in 2,5 ml/kg Olivenöl) ein Leberzellenschaden erzeugt, dessen Ausmaß an der Verlängerung der Hexobarbitalnatrium-Schlafdauer (50 mg/ml/kg i.v.; Schwanzvene, Injektionsdauer 45—60 sec) 47 Stunden nach der $CCl_4$-Gabe bestimmt wird. Die zu prüfenden Verbindungen werden eine Stunde vor der $CCl_4$-Gabe oral in einem Flüssigkeitsvolumen von 10 ml/kg verabreicht.

*Auswertung:*

Der antihepatotoxische Effekt der zu prüfenden Verbindungen (Natrium-Salze in waßriger Lösung) wird an der prozentualen Verkürzung der durch die $CCl_4$-Leberzellschädigung bedingten Schlafdauerverlängerung bei den behandelten Gruppen gegenüber der Schlafdauerverlängerung der $CCl_4$-Kontrollgruppe (=100%) gemessen. Die $ED_{50}$ wurde durch Interpolation aus der Dosis-Wirkungskurve ermittelt.

*Prüfung der antiulcerogenen Wirkung*

Die Ulcusprovokation erfolgt bei 24 h nüchtern gehaltenen Ratten (weiblich, 180—200 g) durch Pylorusligatur (unter Ethernarkose) und orale Applikation von 100 mg/10 ml/kg Acetylsalicylsäure. Die Substanzgabe erfolgt intraduodenal (2,5 ml/kg) unmittelbar nach der Pylorusligatur. Der Wundverschluß wurde mittels Michelklammern vorgenommen. 4 h danach erfolgt die Tötung der Tiere im Etherrausch durch Atlas-Dislokation und die Resektion des Magens. Der längs eröffnete Magen wird auf einer Korplatte fixiert, mit einem Stereomikroskop werden bei 10-facher Vergrößerung Anzahl und Größe (= Durchmesser) vorhandener Ulcera ermittelt. Das Prokukt aus Schweregrad (gemäß nachfolgender Punkteskala) und Anzahl der Ulcera dient als individueller Ulcusindex.

Punkteskala:

| | |
|---|---|
| keine Ulcera | 0 |
| Ø 0,1—1,4 mm | 1 |
| 1,5—2,4 mm | 2 |
| 2,5—3,4 mm | 3 |
| 3,5—4,4 mm | 4 |
| 4,5—5,4 mm | 5 |
| >5,5 mm | 6 |

Als Maß für den antiulcerogenen Effekt dient die Minderung des mittleren Ulcus-Index jeder behandelten Gruppe gegenüber dem der Kontrollgruppe (= 100%).

*Bestimmung der Hemmung der Glucosebildung an der isoliert perfundierten Rattenleber*

Zur Verwendung kommen junge männliche Sprague-Dawley-Ratten (160—200 g). Die Haltung der Tiere erfolgt in Käfigen bis zu 5 Tieren in einem temperierten Raum (23°C) mit festem Tag/Nacht-Rhythmus (12/12 h).

Den Tieren wird 20—22 Stunden vor der Operation das Futter entzogen. Wasseraufnahme erfolgt ad libitum. Operation und Perfusion der Leber erfolgen nach der Technik von R. Scholz et al. [Eur.J. Biochem. 38(1973)64—72]. Als Perfusionsflüssigkeit wird Krebs-Henseleit-Bicarbonat-Puffer (pH 7,4), der mit einer Sauerstoff/Kohlendioxid-Mischung (95/5) gesättigt ist und 1,6 mmol/l L-Lactate und 0,2 mol/l Pyruvat enthält, verwandt. Die Perfusionsflüssigkeit wird in die Leber über eine in die Pfortader eingeführte Kanüle gepumpt. Die austretende Perfusionsflüssigkeit wird über eine in die Vena cava eingeführte Kanüle gesammelt. Die Leber wird ca. 2 Stunden perfundiert. Die Testverbindungen werden von der 32. bis 80. Minute der Perfusion in ansteigenden Konzentrationen (0,02—1.00 mmol/l) je 16 min. infundiert.

Proben der Austrittsperfusionsflüssigkeit werden in Einminutenintervallen gesammelt und auf

Glucose, Lactat und Pyruvat nach enzymatischen Standardmethoden analysiert. Die in Tabelle V angegebenen Prozentwerte beziehen sich auf den vor und nach Zugabe der Verbindungen vorliegenden Zustand, wobei die allein durch Lactat und Pyruvat bedingten Veränderungen gleich 100% gesetzt wurden.

*Bestimmung der Toxizität*

Die Toxizitätsuntersuchungen werden an weiblichen NMRI-Mäusen (Körpergewicht 22—26 g) durchgeführt. Die Tiere (5 Tiere pro Dosis) erhalten Futter und Wasser ad libitum. Verschiedene Dosierungen der Substanzen werden intraperitoneal verabreicht. Die Beobachtungsdauer beträgt 14 Tage. Die $DL_{50}$, d.h. die Dosis, bei der 50% der Tiere sterben, wird aus der Dosiswirkungskurve graphisch ermittelt.

**Patentansprüche fur die Vertragsstaaten: BE, CH, DE, FR, GB, IT, LU, NL, SE**

1. Acylbiphenylylaminoalkansäuren der allgemeinen Formel I

$$R^1—CO—\underset{\underset{A}{|}}{N}—C_nH_{2n}—COOH \quad (I),$$

worin

$R^1$ einen aliphatischen oder alicyclischen Kohlenwasserstoffrest oder einen Phenylrest

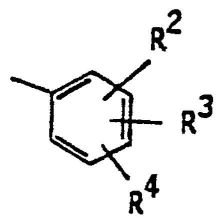

A einen gegebenenfalls hydrierten Biphenylylrest, der durch Halogenatome, Alkyl- oder Alkoxygruppen mit 1 bis 4 Kohlenstoffatomen, gegebenenfalls substituierte Aminogruppen, Hydroxy- oder Nitrogruppen substituiert sein kann,

n eine positive ganze Zahl von 3 bis 5 bedeuten,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe bedeuten,

und ihre Salze mit anorganischen oder organischen Basen.

2. Acylbiphenylylaminoalkansäuren nach Anspruch 1, gekennzeichnet durch die allgemeine Formel I*,

$$R^{1*}—CO—\underset{\underset{A^*}{|}}{N}—C_{n*}H_{2n*}—COOH \quad (I^*),$$

worin

$R^{1*}$ einen aliphatischen Kohlenwasserstoffrest mit 1 bis 7 Kohlenstoffatomen, einen alicyclischen Kohlenwasserstoffrest mit 3 bis 10 Kohlenstoffatomen oder einen Phenylrest

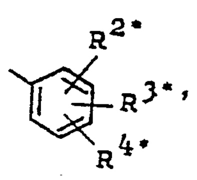

A* eine Gruppe der Formel

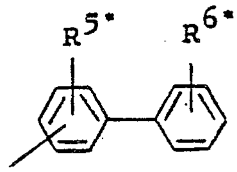, 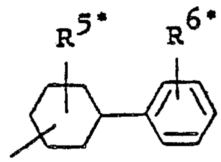 oder 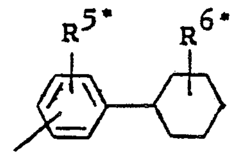

n* eine positive ganze Zahl von 3 bis 5 bedeuten,

$R^{2*}$, $R^{3*}$ und $R^{4*}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe oder eine Trifluormethylgruppe bedeuten,

$R^{5*}$ und $R^{6*}$ gleich oder verschieden sind und ein Wasserstoffatom, ein Halgenatom, eine Methylgruppe, eine Methoxygruppe, eine Hydroxygruppe oder eine Nitrogruppe bedeuten,
und ihre Salze mit anorganischen oder organischen Basen.

3. Acylbiphenylylaminoalkansäuren nach Anspruch 1, gekennzeichnet durch die allgemeine Formel I**,

$$R^{1**}—CO—\underset{\displaystyle A^{**}}{\underset{|}{N}}—C_{n**}H_{2n**}—COOH \qquad (I^{**}),$$

worin

$R^{1**}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylest

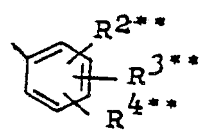

A** eine Gruppe der Formel

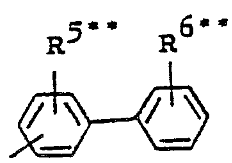 oder 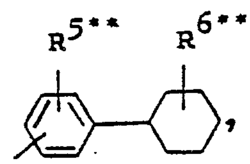,

n** eine positive ganze Zahl von 3 bis 5,

$R^{2**}$ ein Wasserstoffatom bedeuten,

$R^{3**}$ und $R^{4**}$ gleich oder verschieden sind und ein Wasserstoffatom, ein Halogenatom, eine Hydroxygruppe, eine Methoxygruppe, eine Methylgruppe, eine Aklanoyloxygruppe mit 2 bis 5 Kohlenstoffatomen, eine Nitrogruppe oder eine Trifluormethylgruppe bedeuten,

einer der Substituenten $R^{5**}$ oder $R^{6**}$ ein Wasserstoffatom und der andere ein Wasserstoffatom, ein Halogenatom, eine Methylgruppe, eine Methoxygruppe, eine Hydroxygruppe oder eine Nitrogruppe bedeutet,
und ihre Salze mit anorganischen oder organischen Basen.

4. Acylbiphenylylaminoalkansäuren nach Anspruch 1, gekennzeichnet durch die allgemeine Formel I***,

$$R^{1***}—CO—\underset{\displaystyle A^{***}}{\underset{|}{N}}—C_{n***}H_{2n***}—COOH \qquad (I^{***}),$$

worin

$R^{1***}$ einen aliphatischen Kohlenwasserstoffrest mit 1 bis 3 Kohlenstoffatomen oder einen Phenylrest

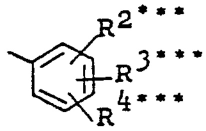,

A*** eine Gruppe der Formel

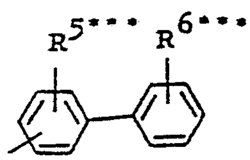

oder

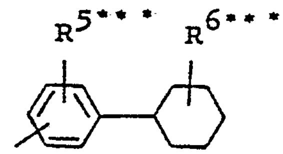

n*** eine positive ganze Zahl von 3 bis 5,

$R^{2***}$ ein Wasserstoffatom bedeuten,

$R^{3***}$ und $R^{4***}$ gleich oder verschieden sind und ein Wasserstoffatom, ein Fluoratom, ein Chloratom, eine Hydroxygruppe, eine Methoxygruppe oder eine Trifluormethylgruppe bedeuten,

einer der Substituenten $R^{5***}$ oder $R^{6***}$ ein Wasserstoffatom und der andere ein Wasserstoffatom, oder eine Methoxygruppe bedeutet,

und ihre Salze mit anorganischen oder organischen Basen.

5. Acylbiphenylylaminoalkansäuren nach Anspruch 1, gekennzeichnet durch die allgemeine Formel I****

$$R^{1****}\text{—CO—}\underset{\displaystyle A^{****}}{\underset{|}{N}}\text{—}C_{n****}H_{2n****}\text{—COOH} \qquad (I^{****}),$$

worin

$R^{1****}$ eine Alkylgruppe mit 1 bis 3 Kohlenstoffatomen, einen Alkenylrest mit 2 bis 3 Kohlenstoffatomen, oder eine Phenylgruppe

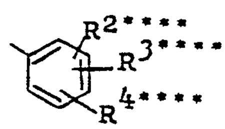

A**** eine Gruppe der Formel

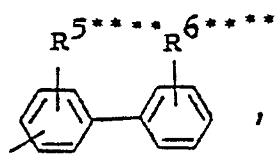,

n**** eine positive ganze Zahl von 3 bis 5,

$R^{2****}$ ein Wasserstoffatom,

$R^{3****}$ ein Wasserstoffatom, ein Fluoratom, ein Chloratom, eine Hydroxygruppe, eine Methoxygruppe oder Trifluormethylgruppe,

$R^{4****}$ ein Wasserstoffatom oder ein Chloratom,

einer der Substituenten $R^{5****}$ oder $R^{6****}$ ein Wasserstoffatom und der andere ein Wasserstoffatom oder eine Methoxygruppe bedeutet.

und ihre pharmakologisch verträglichen Salze mit anorganischen und organischen Basen.

6. Verbindungen nach Anspruch 4 oder 5, in denen A*** bzw. A**** die Bedeutung eines 2-Biphenylylrestes hat, und ihre pharmakologisch verträglichen Salze mit anorganischen oder organischen Basen.

7. Verbindungen nach Anspruch 5, in denen $R^{3****}$ ein Fluoratom, ein Chloratom, eine Hydroxygruppe oder eine Methoxygruppe bedeutet und $R^{5****}$ und $R^{6****}$ Wasserstoffatome darstellen, sowie ihre pharmakologisch verträglichen Salze mit anorganischen und organischen Basen.

8. Verbindungen nach Anspruch 5, in denen A**** einen 2-Biphenylylrest darstellt, $R^{3****}$ ein Fluoratom, ein Chloratom, eine Hydroxygruppe oder eine Methoxygruppe bedeutet und $R^{5****}$ und $R^{6****}$ Wasserstoffatome darstellen, sowie ihre pharmakologisch verträglichen Salze mit anorganischen und organischen Basen.

9. Arzneimittel, enthaltend eine oder mehrere Verbindungen nach einem oder mehreren der Ansprüche 1 bis 8 und/oder ihre pharmakologisch verträglichen Salze mit anorganischen oder organischen Basen.

10. Verfahren zur Herstellung der Acylbiphenylylaminoalkansäuren der allgemeinen Formel I

$$R^1—CO—N(A)—C_nH_{2n}—COOH \quad (I),$$

sowie ihrer Salze mit anorganischen und organischen Basen, dadurch gekennzeichnet, daß man

a) eine Biphenylylaminoalkansäure der allgemeinen Formel II

$$A—NH—C_nH_{2n}—COOH \quad (II),$$

gegebenenfalls unter Schutz der Carboxylgruppe, mit einem Acylderivat der allgemeinen Formel III

$$R^1—CO—R^7 \quad (III),$$

worin $R^7$ eine Fluchtgruppe oder eine $R^1—CO—O$-Gruppe darstellt acyliert und gewünschtenfalls anschließend in die Salze überführt oder

b) eine Biphenylylaminoalkensäure der allgemeinen Formel IV

$$R^1—CO—N(A)—C_nH_{2n-2}—COOH \quad (IV),$$

gegebenenfalls unter Schutz der Carboxylgruppe, hydriert, und gewünschtenfalls anschließend in die Salze überführt oder

c) ein funktionelles Acylbiphenylylaminoalkansäurederivat der allgemeinen Formel V

$$R^1—CO—N(A)—C_nH_{2n}—G \quad (V),$$

worin G ein funktionelles Derivat einer Carboxylgruppe bedeutet, solvolysiert und gewünschtenfalls anschließend in die Salze überführt, wobei in den Formeln I bis V die Symbole $R^1$, A und n die in Anspruch 1 angegebene Bedeutung haben.

11. Verfahren nach Anspruch 10 zur Herstellung der Acylbiphenylylaminoalkansäure der allgemeinen Formel I*

$$R^{1*}—CO—N(A^*)—C_{n*}H_{2n*}—COOH \quad (I^*),$$

sowie ihrer Salze mit anorganischen oder organischen Basen, dadurch gekennzeichnet, daß man

a) eine Biphenylylaminoalkansäure der allgemeinen Formel II*

$$A^*—NH—C_nH_{2n*}—COOH \quad (II^*),$$

gegebenenfalls unter Schutz der Carboxylgruppe, mit einem Acylderivat der allgemeinen Formel III*

$$R^{1*}—CO—R^{7*} \quad (III^*),$$

worin $R^{7*}$ eine Fluchtgruppe oder eine $R^{1*}—CO—O$-Gruppe darstellt acyliert und gewünschtenfalls anschließend in die Salze überführt oder

b) eine Biphenylylaminoalkensäure der allgemeinen Formel IV*

$$R^{1*}—CO—\underset{\substack{|\\ A^*}}{N}—C_{n*}H_{2n*-2}—COOH \qquad (IV^*),$$

gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V*

$$R^{1*}—CO—\underset{\substack{|\\ A^*}}{N}—C_{n*}H_{2n*}—G^* \qquad (V^*),$$

worin G* ein funktionelles Derivat einer Carboxylgruppe bedeutet, solvolysiert und gewünschtenfalls anschließend in die Salze überführt, wobei in den Formeln I* bis V* die Symbole $R^{1*}$, A* und n* die in Anspruch 2 angegebenen Bedeutungen haben.

12. Verfahren nach Anspruch 10 zur Herstellung der Acylbiphenylylaminoalkansäuren der allgemeinen Formel I**

$$R^{1**}—CO—\underset{\substack{|\\ A^{**}}}{N}—C_{n**}H_{2n**}—COOH \qquad (I^{**}),$$

sowie ihrer Salze mit anorganischen oder organischen Basen, dadurch gekennzeichnet, daß man

a) eine Biphenylylaminoalkansäure der allgemeinen Formel II**

$$A^{**}—NH—C_{n**}H_{2n**}—COOH \qquad (II^{**}),$$

gegebenenfalls unter Schutz der Carboxylgruppe, mit einem Acylderivat der allgemeinen Formel III**

$$R^{1**}—CO—R^{7**} \qquad (III^{**}),$$

worin $R^{7**}$ eine Fluchtgruppe oder eine $R^{1**}$—CO—O-Gruppe darstellt, acyliert und gewünschtenfalls anschließend in die Salze überführt oder

b) eine Biphenylylaminoalkensäure der allgemeinen Formel IV**

$$R^{1**}—CO—\underset{\substack{|\\ A^{**}}}{N}—C_{n**}H_{2n**-2}—COOH \qquad (IV^{**}),$$

gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V**

$$R^{1**}—CO—\underset{\substack{|\\ A^{**}}}{N}—C_{n**}H_{2n**}—G^{**} \qquad (V^{**}),$$

worin G** ein funktionelles Derivat einer Carboxylgruppe bedeutet, solvolysiert und gewünschtenfalls anschließend in die Salze überführt, wobei in den Formeln I** bis V** die Symbole $R^{1**}$, A** und n** die in Anspruch 3 angegebenen Bedeutungen haben.

13. Verfahren nach Anspruch 10 zur Herstellung der Acylbiphenylylaminoalkansäuren der allgemeinen Formel I***

$$R^{1***}—CO—\underset{\substack{|\\ A^{***}}}{N}—C_{n***}H_{2n***}—COOH \qquad (I^{***}),$$

sowie ihrer Salze mit anorganischen und organischen Basen, dadurch gekennzeichnet, daß man

a) eine Biphenylylaminoalkansäure der allgemeinen Formel II***

$$A^{***}—NH—C_{n^{***}}H_{2n^{***}}—COOH \quad (II^{***}),$$

gegebenenfalls unter Schutz der Carboxylgruppe, mit einem Acylderivat der allgemeinen Formel III***

$$R^{1***}—CO—R^{7***} \quad (III^{***}),$$

worin $R^{7***}$ eine Fluchtgruppe oder eine $R^{1***}$—CO—O-Gruppe darstellt, acyliert und gewünschtenfalls anschließend in die Salze überführt oder

b) eine Biphenylylaminoalkensäure der allgemeinen Formel IV***

$$R^{1***}—CO—\underset{\underset{A^{***}}{|}}{N}—C_{n^{***}}H_{2n^{***}-2}—COOH \quad (IV^{***}),$$

gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V***

$$R^{1***}—CO—\underset{\underset{A^{***}}{|}}{N}—C_{n^{***}}H_{2n^{***}}—G^{***} \quad (V^{***}),$$

worin G*** ein funktionelles Derivat einer Carboxylgruppe bedeutet, solvolysiert und gewünschtenfalls anschließend in die Salze überführt, wobei in den Formeln I*** bis V*** die Symbole $R^{1***}$, A*** und n*** die in Anspruch 4 angegebenen Bedeutungen haben.

14. Verfahren nach Anspruch 10 zur Herstellung der Acylbiphenylylaminoalkansäuren der allgemeinen Formel I****

$$R^{1****}—CO—\underset{\underset{A^{****}}{|}}{N}—C_{n^{****}}H_{2n^{****}}—COOH \quad (I^{****}),$$

sowie ihrer pharmakologisch verträglichen Salze mit anorganischen und organischen Basen, dadurch gekennzeichnet, daß man

a) eine Biphenylylaminoalkansäure der allgemeinen Formel II****

$$A^{****}—NH—C_{n^{****}}H_{2n^{****}}—COOH \quad (II^{****}),$$

gegebenenfalls unter Schutz der Carboxylgruppe, mit einem Acylderivat der allgemeinen Formel III****

$$R^{1****}—CO—R^{7****} \quad (III^{****}),$$

worin $R^{7****}$ eine Fluchtgruppe oder eine $R^{1****}$—CO—O-Gruppe darstellt acyliert und gewünschtenfalls anschließend in die Salze überführt oder

b) eine Biphenylylaminoalkensäure der allgemeinen Formel IV****

$$R^{1****}—CO—\underset{\underset{A^{****}}{|}}{N}—C_{n^{****}}H_{2n^{****}-2}—COOH \quad (IV^{****}),$$

gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V****

$$R^{1****}—CO—\underset{\underset{A^{****}}{|}}{N}—C_{n^{****}}H_{2n^{****}}—G^{****} \quad (V^{****}),$$

worin G**** ein funktionelles Derivat einer Carboxylgruppe bedeutet, solvolysiert und gewünschtenfalls anschließend in die Salze überführt, wobei in den Formeln I**** bis V**** die Symbole $R^{1****}$, A**** und n**** die in Anspruch 5 angegebenen Bedeutungen haben.

15. Verbindungen gemäß einem oder mehreren der Anspruch 1 bis 8 zur Behandlung und Prophylaxe von Krankheiten, die auf Erkrankungen des Magens oder Darms oder auf Minderleistungen von Pankreas, Galle und/oder Leber beruhen.

**Patentansprüche für den Vertraagstaat: AT**

1. Verfahren zur Herstellung der Acylbiphenylylaminoalkansäuren der allgemeinen Formel I

$$R^1\text{—CO—}\underset{\substack{|\\ A}}{N}\text{—}C_nH_{2n}\text{—COOH} \qquad (I),$$

worin

$R^1$ einen aliphatischen oder alicyclischen Kohlenwasserstoffrest oder einen Phenylrest

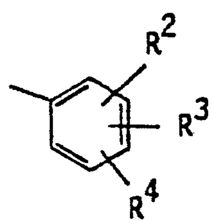

A einen gegebenenfalls hydrierten Biphenylylrest, der durch Halogenatome, Alkyl- oder Alkoxygruppen mit 1 bis 4 Kohlenstoffatomen, gegebenenfalls substituierte Aminogruppen, Hydroxy- oder Nitrogruppen substituiert sein kann,

n eine positive ganze Zahl von 3 bis 5 bedeuten,

$R^2$, $R^3$ und $R^4$ gleich oder verschieden sind und ein Wasserstoffatom ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluoromethylgruppe bedeuten.

sowie ihrer Salze mit anorganischen und organischen Basen, dadurch gekennzeichnet, daß man

a) eine Biphenylylaminoalkansäure der allgemeinen Formel II

$$A\text{—NH—}C_nH_{2n}\text{—COOH} \qquad (II),$$

worin A und n die vorstehend angegebene Bedeutung haben, gegebenenfalls unter Schutz der Carboxylgruppe, mit einem Acylderivat der allgemeinen Formel III

$$R^1\text{—CO—}R^7 \qquad (III),$$

worin $R^7$ eine Fluchtgruppe oder eine $R^1$—CO—O-Gruppe darstellt und $R^1$ die oben angegebene Bedeutung hat, acyliert und gewünschtenfalls anschließend in die Salze überführt oder

b) eine Biphenylylaminoalkensäure der allgemeinen Formel IV

$$R^1\text{—CO—}\underset{\substack{|\\ A}}{N}\text{—}C_nH_{2n-2}\text{—COOH} \qquad (IV),$$

worin $R^1$, A und n die vorstehend angegebene Bedeutung haben, gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V

$$R^1\text{—CO—}\underset{\substack{|\\ A}}{N}\text{—}C_nH_{2n}\text{—G} \qquad (V),$$

worin $R^1$, A und n die oben angegebene Bedeutung haben und G ein funktionelles Derivat einer Carboxylgruppe bedeutet, solvolysiert und gewünschtenfalls anschließend in die Salze überführt.

2. Verfaahren nach Anspruch 1 zur Herstellung der Acylbiphenylylaminoalkansäure der allgemeinen Formel I*

$$R^{1*}-CO-\underset{\displaystyle A^*}{\underset{|}{N}}-C_{n*}H_{2n*}-COOH \qquad (I^*),$$

worin

$R^{1*}$ einen aliphatischen Kohlenwasserstoffrest mit 1 bis 7 Kohlenstoffatomen, einen alicyclischen Kohlenwasserstoffrest mit 3 bis 10 Kohlenstoffatomen oder einen Phenylrest

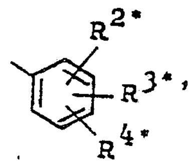

A* eine Gruppe der Formel

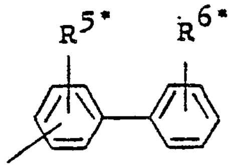 , 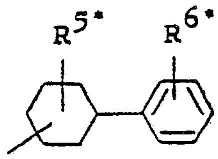 oder 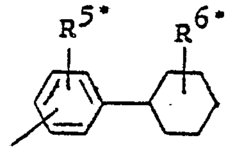

n* eine positive ganze Zahl von 3 bis 5 bedeuten,

$R^{2*}$, $R^{3*}$ und $R^{4*}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe oder eine Trifluormethylgruppe bedeuten,

$R^{5*}$ und $R^{6*}$ gleich oder verschieden sind und ein Wasserstoffatom, ein Halogenatom, eine Methylgruppe, eine Methoxygruppe, eine Hydroxygruppe oder eine Nitrogruppe bedeuten,

sowie ihrer Salze mit anorganischen oder organischen Basen, dadurch gekennzeichnet, daß man

a) eine Biphenylylaminoalkansäure der allgemeinen Formel II*

$$A^*-NH-C_{n*}H_{2n*}-COOH \qquad (II^*),$$

worin A* und n* die vorstehend angegebene Bedeutung haben, gegebenenfalls unter Schutz der Carboxylgruppe, mit einem Acylderivat er allgemeinen Formel III*

$$R^{1*}-CO-R^{7*} \qquad (III^*),$$

worin $R^{7*}$ eine Fluchtgruppe oder eine $R^{1*}$—CO—O-Gruppe darstellt und $R^{1*}$ die oben angegebene Bedeutung hat, acyliert und gewünschtenfalls anschließend in die Salze überführt oder

b) eine Biphenylylaminoalkensäure der allgemeinen Formel IV*

$$R^{1*}-CO-\underset{\displaystyle A^*}{\underset{|}{N}}-C_{n*}H_{2n*-2}-COOH \qquad (IV^*),$$

worin $R^{1*}$, A* und n* die vorstehend angegebene Bedeutung haben, gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V*

$$R^{1*}-CO-\underset{\displaystyle A^*}{\underset{|}{N}}-C_{n*}H_{2n*}-G^* \qquad (V^*),$$

worin $R^{1*}$, A* und n* die oben angegebene Bedeutung haben und G* ein funktionelles Derivat einer Carboxylgruppe bedeutet, solvolysiert und gewünschtenfalls anschließend in die Salze überführt.

3. Verfahren nach Anspruch 1 zur Herstellung der Acylbiphenylylaminoalkansäuren der allgemeinen Formel I**

$$R^{1**}-CO-\underset{\displaystyle A^{**}}{\underset{|}{N}}-C_{n**}H_{2n**}-COOH \qquad (I^{**}),$$

worin

$R^{1**}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylrest

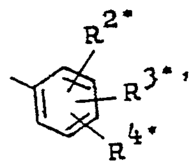,

A** eine Gruppe der Formel

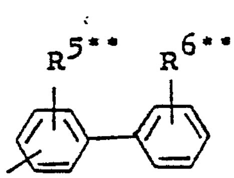

oder

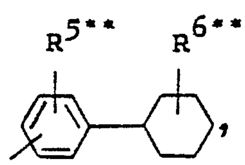,

n** eine positive ganze Zahl von 3 bis 5,

$R^{2**}$ ein Wasserstoffatom bedeuten,

$R^{3**}$ und $R^{4**}$ gleich oder verschieden sind und ein Wasserstoffatom, ein Halogenatom, eine Hydroxygruppe, eine Methoxygruppe, eine Methylgruppe eine Alkanoyloxygruppe mit 2 bis 5 Kohlenstoffatomen, eine Nitrogruppe oder eine Trifluormethylgruppe bedeuten.

einer der Substituenten $R^{5**}$ oder $R^{6**}$ ein Wasserstoffatom und der andere ein Wasserstoffatom, ein Halogenatom, eine Methylgruppe, eine Methoxygruppe, eine Hydroxygruppe oder eine Nitrogruppe bedeutet,

sowie ihrer Salze mit anorganischen oder organischen Basen, dadurch gekennzeichnet, daß man

a) eine Biphenylylaminoalkansäure der allgemeinen Formel II**

$$A^{**}-NH-C_{n**}H_{2n**}-COOH \qquad (II^{**}),$$

worin A** und n** die vorstehend angegebene Bedeutung haben, gegebenenfalls unter Schutz der Carboxylgruppe, mit einem Acylderivat der allgemeinen Formel III**

$$R^{1**}-CO-R^{7**} \qquad (III^{**}),$$

worin $R^{7**}$ eine Fluchtgruppe oder eine $R^{1**}$—CO—O-Gruppe darstellt und $R^{1**}$ die oben angegebene Bedeutung hat, acyliert und gewünschtenfalls anschließend in die Salze überführt oder

b) eine Biphenylylaminoalkensäure der allgemeinen Formel IV**

$$R^{1**}-CO-\underset{\displaystyle A^{**}}{\underset{|}{N}}-C_{n**}H_{2n**-2}-COOH \qquad (IV^{**}),$$

worin $R^{1**}$, A** und n** die vorstehend angegebene Bedeutung haben, gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel V**

$$R^{1**}-CO-\underset{\displaystyle A^{**}}{\underset{|}{N}}-C_{n**}H_{2n**}-G^{**} \qquad (V^{**}),$$

worin $R^{1**}$, $A^{**}$ und $n^{**}$ die oben angegebene Bedeutung haben und $G^{**}$ ein funktionelles Derivat einer Carboxylgruppe bedeutet, solvolysiert und gewünschtenfalls anschließend in die Salze überführt.

4. Verfahren nach Anspruch 1 zur Herstellung der Acylbiphenylylaminoalkansäuren der allgemeinen Formel $I^{***}$

$$R^{1***}—CO—\underset{\displaystyle A^{***}}{\underset{|}{N}}—C_{n***}H_{2n***}—COOH \qquad (I^{***}),$$

worin

$R^{1***}$ einen aliphatischen Kohlenwasserstoffrest mit 1 bis 3 Kohlenstoffatomen oder einen Phenylrest

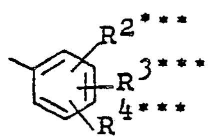

$A^{***}$ eine Gruppe der Formel

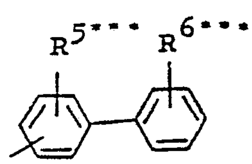

oder

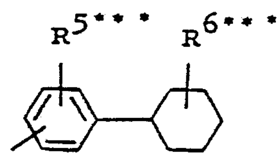

$n^{***}$ eine positive ganze Zahl von 3 bis 5,

$R^{2***}$ ein Wasserstoffatom bedeuten,

$R^{3***}$ und $R^{4***}$ gleich oder verschieden sind und ein Wasserstoffatom, ein Fluoratom, ein Chloratom, ein Hydroxygruppe, eine Methoxygruppe oder eine Trifluormethylgruppe bedeuten,

einer der Substituenten $R^{5***}$ oder $R^{6***}$ ein Wasserstoffatom und der andere ein Wasserstoffatom oder eine Methoxygruppe bedeutet,

sowie ihrer Salze mit anorganischen und organischen Basen, dadurch gekennzeichnet, daß man

a) eine Biphenylylaminoalkansäure der allgemeinen Formel $II^{***}$

$$A^{***}—NH—C_{n***}H_{2n***}—COOH \qquad (II^{***}),$$

worin $A^{***}$ und $n^{***}$ die vorstehend angegebene Bedeutung haben, gegebenenfalls unter Schutz der Carboxylgruppe, mit einem Acylderivat der allgemeinen Formel $III^{***}$

$$R^{1***}—CO—R^{7***} \qquad (III^{***}),$$

worin $R^{7***}$ eine Fluchtgruppe oder eine $R^{1***}$—CO—O-Gruppe darstellt und $R^{1***}$ die oben angegebene Bedeutung hat, acyliert und gewünschtenfalls anschließend in die Salze überführt oder

b) eine Biphenylylaminoalkensäure der allgemeinen Formel $IV^{***}$

$$R^{1***}—CO—\underset{\displaystyle A^{***}}{\underset{|}{N}}—C_{n***}H_{2n***-2}—COOH \qquad (IV^{***}),$$

worin $R^{1***}$, $A^{***}$ und $n^{***}$ die vorstehend angegebene Bedeutung haben, gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel $V^{***}$

$$R^{1***}—CO—\underset{\displaystyle A^{***}}{\underset{|}{N}}—C_{n***}H_{2n***}—G^{***} \qquad (V^{***}),$$

worin $R^{1***}$, $A^{***}$ und $n^{***}$ die oben angegebene Bedeutung haben und $G^{***}$ ein funktionelles Derivat einer Carboxylgruppe bedeutet, solvolysiert und gewünschtenfalls anschließend in die Salze überführt.

5. Verfahren nach Anspruch 1 zur Herstellung der Acylbiphenylylaminoalkansäuren der allgemeinen Formel $I^{****}$,

$$R^{1****}—CO—\underset{\displaystyle A^{****}}{\underset{|}{N}}—C_{n^{****}}H_{2n^{****}}—COOH \qquad (I^{****})$$

worin

$R^{1****}$ eine Alkylgruppe mit 1 bis 3 Kohlenstoffatomen, einen Alkenylrest mit 2 bis 3 Kohlenstoffatomen, oder eine Phenylgruppe

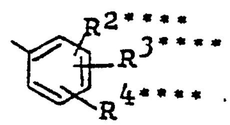 .

$A^{****}$ eine Gruppe der Formel

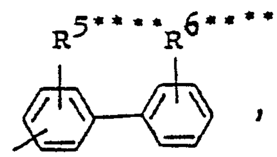 ,

$n^{****}$ eine ganze positive Zahl von 3 bis 5,

$R^{2****}$ ein Wasserstoffatom,

$R^{3****}$ ein Wasserstoffatom, ein Fluoratom, ein Chloratom, eine Hydroxygruppe, eine Methoxygruppe oder Trifluormethylgruppe,

$R^{4****}$ ein Wasserstoffatom oder ein Chloratom,

einer der Substituenten $R^{5****}$ oder $R^{6****}$ ein Wasserstoffatom und der andere ein Wasserstoffatom oder eine Methoxygruppe bedeutet.

sowie ihrer pharmakologisch verträglichen Salze mit anorganischen und organischen Basen, dadurch gekennzeichnet, daß man

a) eine Biphenylylaminoalkansäure der allgemeinen Formel $II^{****}$

$$A^{****}—NH—C_{n^{****}}H_{2n^{****}}—COOH \qquad (II^{****}),$$

worin $A^{****}$ und $n^{****}$ die vorstehend angegebene Bedeutung haben, gegebenenfalls unter Schutz der Carboxylgruppe, mit einem Acylderivat der allgemeinen Formel $III^{****}$

$$R^{1****}—CO—R^{7****} \qquad (III^{****}),$$

worin $R^{7****}$ eine Fluchtgruppe oder eine $R^{1****}—CO—O$-Gruppe darstellt und $R^{1****}$ die oben angegebene Bedeutung hat, acyliert und gewünschtenfalls anschließend in die Salze überführt oder

b) eine Biphenylylaminoalkensäure der allgemeinen Formel $IV^{****}$

$$R^{1****}—CO—\underset{\displaystyle A^{****}}{\underset{|}{N}}—C_{n^{****}}H_{2n^{****}-2}—COOH \qquad (IV^{****}),$$

worin $R^{1****}$, $A^{****}$ und $n^{****}$ die vorstehend angegebene Bedeutung haben, gegebenenfalls unter Schutz der Carboxylgruppe, hydriert und gewünschtenfalls anschließend in die Salze überführt oder

c) ein funktionelles Acylbiphenylylaminoalkansäurederivat der allgemeinen Formel $V^{****}$

$$R^{1****}—CO—\underset{\displaystyle A^{****}}{\underset{|}{N}}—C_{n^{****}}H_{2n^{****}}—G^{****} \qquad (V^{****}),$$

worin $R^{1****}$, $A^{****}$ und $n^{****}$ die oben angegebene Bedeutung haben und $G^{****}$ ein funktionelles Derivat einer Carboxylgruppe bedeutet, solvolysiert und gewünschtenfalls anschließend in die Salze überführt.

6. Verfahren nach Anspruch 4 oder 5, wobei $A^{***}$ bzw. $A^{****}$ die Bedeutung eines 2-Biphenylylrestes hat.

7. Verfahren nach Anspruch 5, wobei $R^{3****}$ ein Fluoratom, ein Chloratom, eine Hydroxygruppe oder eine Methoxygruppe bedeutet und $R^{5****}$ und $R^{6****}$ Wasserstoffatome darstellen.

8. Verfahren nach Anspruch 5, wobei $A^{****}$ einen 2-Biphenylylrest darstellt, $R^{3****}$ ein Fluoratom, ein Chloratom, eine Hydroxygruppe oder eine Methoxygruppe bedeutet und $R^{5****}$ und $R^{6****}$ Wasserstoffatome darstellen.

9. Verfahren nach einem oder mehreren der Ansprüche 1 bis 8, wobei die Fluchtgruppe $R^7$, $R^{7*}$, $R^{7**}$, $R^{7***}$ bzw. $R^{7****}$ ein Chlor- oder Brom- atom und G, $G^*$, $G^{**}$, $G^{***}$ bzw. $G^{****}$ eine Nitril-, Carbonsäureester- oder Carbonsäureamidgruppe darstellt.

**Revendications pour les Etats contractants: BE, CH, DE, FR, GB, IT, LU, NL, SE,**

1. Acides acyl-biphénylylamino-alcanoiques de formule générale I:

$$R^1\text{—CO—}\underset{\displaystyle A}{\underset{|}{N}}\text{—}C_nH_{2n}\text{—COOH} \qquad (I)$$

dans laquelle

$R^1$ représente un radical d'hydrocarbure aliphatique ou alicyclique ou un group phényle

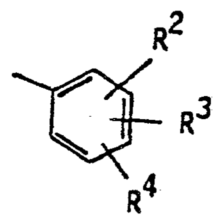

A représente un groupe biphénylyle éventuellement hydrogéné et éventuellement substitué par des atomes d'halogène, des groupes alkyle ou alcoxy contenant 1 à 4 atomes de carbone, des groupes amino éventuellement substitués, des groupes hydroxy ou des groupes nitro,

n est un nombre positif entier d'une valeur comprise entre 3 et 5,

$R^2$, $R^3$ et $R^4$ sont identiques ou différents et représentent un atome d'hydrogène, un atome d'halogène, un groupe alkyle, un groupe hydroxy, un groupe alcoxy, un groupe acyloxy, un groupe amino éventuellement substitué, un groupe nitro ou un groupe trifluorométhyle, ainsi que leurs sels avec des bases organiques ou inorganiques.

2. Acides acyl-biphénylylamino-alcanoiques selon la revendication 1, caractérisés en ce qu'ils répondent à la formule générale I*:

$$R^{1*}\text{—CO—}\underset{\displaystyle A^*}{\underset{|}{N}}\text{—}C_{n*}H_{2n*}\text{—COOH} \qquad (I^*)$$

dans laquelle

$R^{1*}$ représente un radical d'hydrocarbure aliphatique contenant 1 à 7 atomes de carbone, un radical d'hydrocarbure alicyclique contenant 3 à 10 atomes de carbone ou un groupe phényle

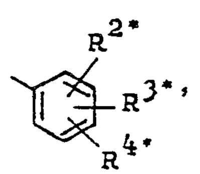

$A^*$ représente un groupe de formule:

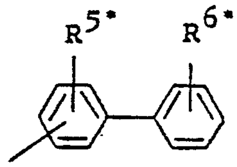, 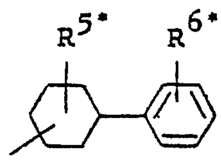 ou 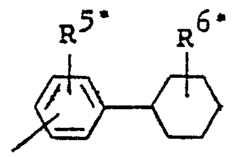

$n^*$ est un nombre positif entier d'une valeur comprise entre 3 et 5,

$R^{2*}$, $R^{3*}$ et $R^{4*}$ sont identiques ou différents et représentent chacun un atome d'hydrogène, un atome d'halogène, un groupe hydroxy, un groupe alkyle contenant 1 à 4 atomes de carbone, un groupe alcoxy contenant 1 à 4 atomes de carbone, un groupe alcanoyloxy contenant 2 à 5 atomes de carbone, un groupe nitro ou un groupe trifluorométhyle,

$R^{5*}$ et $R^{6*}$ sont identiques ou différents et représentent chacun un atome d'hydrogène, un atome d'halogène, un groupe méthyle, un groupe méthoxy, un groupe hydroxy ou un groupe nitro, ainsi que leurs sels avec des bases organiques ou inorganiques.

3. Acides acyl-biphénylylamino-alcanoiques selon la revendication 1, caractérisés en ce qu'ils répondent à la formule générale I**:

$$R^{1**}\text{—CO—}\underset{\displaystyle A^{**}}{\underset{|}{N}}\text{—}C_{n^{**}}H_{2n^{**}}\text{—COOH} \qquad (I^{**})$$

dans laquelle

$R^{1**}$ représente un radical d'hydrocarbure aliphatique contenant 1 à 5 atomes de carbone, un radical d'hydrocarbure alicyclique contenant 5 à 7 atomes de carbone ou un groupe phényle

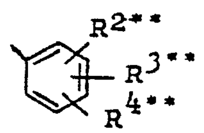

$A^{**}$ représente un groupe de formule:

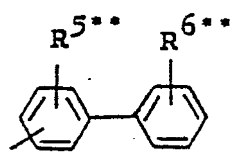 ou 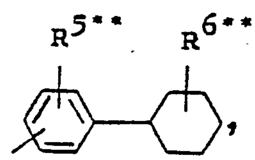,

$n^{**}$ est un nombre positif entier d'une valeur comprise entre 3 et 5,

$R^{2**}$ représente un atome d'hydrogène,

$R^{3**}$ et $R^{4**}$ sont identiques ou différents et représentent chacun un atome d'hydrogène, ou atome d'halogène, un groupe hydroxy, un groupe méthoxy, un groupe méthyle, un groupe alcanoyloxy contenant 2 à 5 atomes de carbone, un groupe nitro ou un groupe trifluorométhyle,

un des substituants $R^{5**}$ et $R^{6**}$ représentant un atome d'hydrogène et l'autre, un atome d'hydrogène, un atome d'halogène, un groupe méthyle, un groupe méthoxy, un groupe hydroxy ou un groupe nitro,

ainsi que leurs sels avec des bases organiques ou inorganiques.

4. Acides acyl-biphénylylamino-alcanoiques selon la revendication 1, caractérisés en ce qu'ils répondent à la formule générale I***:

$$R^{1***}\text{—CO—}\underset{\displaystyle A^{***}}{\underset{|}{N}}\text{—}C_{n^{***}}H_{2n^{***}}\text{—COOH} \qquad (I^{***})$$

dans laquelle

$R^{1***}$ représente un radical d'hydrocarbure aliphatique contenant 1 à 3 atomes de carbone ou un groupe phényle

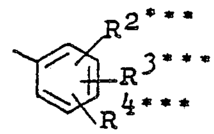

A*** représente un groupe de formule:

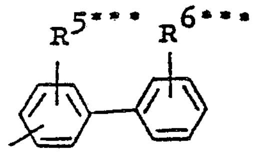

ou

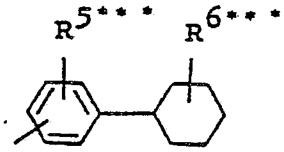

n*** est un nombre positif entier d'une valeur comprise entre 3 et 5,

$R^{2***}$ représente un atome d'hydrogène,

$R^{3***}$ et $R^{4***}$ sont identiques ou différents et représentent chacun un atome d'hydrogène, un atome de fluor, un atome de chlore, un groupe hydroxy, un groupe méthoxy ou un groupe trifluorométhyle,

un des substituants $R^{5***}$ et $R^{6***}$ est un atome d'hydrogène et l'autre est un atome d'hydrogène ou un groupe méthoxy,

ainsi que leurs sels avec des bases organiques ou inorganiques.

5. Acides acyl-biphénylylamino-alcanoiques selon la revendication 1, caractérisés en ce qu'ils répondent à la formule générale I****:

$$R^{1****}\text{—CO—}\underset{\substack{|\\ A^{****}}}{N}\text{—}C_{n****}H_{2n****}\text{—COOH} \qquad (I^{****})$$

dans laquelle

$R^{1****}$ représente un groupe alkyle contenant 1 à 3 atomes de carbone, un groupe alcényle contenant 2 ou 3 atomes de carbone, ou un groupe phényle:

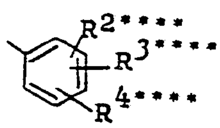

A**** représente un groupe de formule:

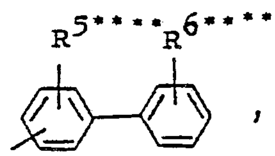

n**** est un nombre positif entier d'une valeur comprise entre 3 et 5,

$R^{2****}$ représente un atome d'hydrogène,

$R^{3****}$ représente un atome d'hydrogène, un atome de fluor, un atome de chlore, un groupe hydroxy, un groupe méthoxy ou un groupe trifluorométhyle,

$R^{4****}$ représente un atome d'hydrogène ou un atome de chlore,

un des substituants $R^{5****}$ et $R^{6****}$ est un atome d'hydrogène, tandis que l'autre est un atome d'hydrogène ou un groupe méthoxy,

ainsi que leurs sels pharmacologiquement acceptables avec des bases organiques ou inorganiques.

6. Composés selon les revendications 4 et 5, dans lesquels A*** ou A**** représente un radical 2-biphénylyle, ainsi que leurs sels pharmacologiquement acceptables avec des bases organiques ou inorganiques.

7. Composés selon la revendication 5, dans lesquels $R^{3****}$ représente un atome de fluor, un atome de chlore, un groupe hydroxy ou un groupe méthoxy, tandis que $R^{5****}$ et $R^{6****}$ représentent

chacun un atome d'hydrogène, ainsi que leurs sels pharmacologiquement acceptables avec des bases organiques ou inorganiques.

8. Composés selon la revendication 5, dans lesquels A**** représente un radical 2-biphénylyle, $R^{3****}$ représente un atome de fluor, un atome de chlore, un groupe hydroxy ou un groupe méthoxy, tandis que $R^{5****}$ et $R^{6****}$ représentent chacun un atome d'hydrogène, ainsi que leurs sels pharmacologiquement acceptables avec des bases organiques ou inorganiques.

9. Médicaments contenant un ou plusieurs composés selon une ou plusieurs des revendications 1 à 8 et/ou leurs sels pharmacologiquement acceptables avec des bases inorganiques ou organiques.

10. Procédé de préparation d'acides acyl-biphénylylamino-alcanoiques de formule générale I:

$$R^1—CO—N(A)—C_nH_{2n}—COOH \quad (I)$$

ainsi que de leurs sels avec des bases organiques ou inorganiques, caractérisé en ce que:

a) on acyle un acide biphénylylamino-alcanoïque de formule générale II:

$$A—NH—C_nH_{2n}—COOH \quad (II)$$

en protégeant éventuellement le groupe carboxy, avec un dérivé acyle de formule générale III:

$$R^1—CO—R^7 \quad (III)$$

dans laquelle $R^7$ représente un groupe qui s'éloigne ou un groupe $R^1—CO—O—$, puis on le transforme éventuellement en un sel, ou

b) on hydrogène un acide biphénylylamino-alcénoïque de formule générale IV:

$$R^1—CO—N(A)—C_nH_{2n-2}—COOH \quad (IV)$$

en protégeant éventuellement le groupe carboxy, puis on le transforme éventuellement en un sel, ou

c) on solvolyse un dérivé fonctionnel d'un acide acylbiphénylylamino-alcanoïque de formule générale V:

$$R^1—CO—N(A)—C_nH_{2n}—G \quad (V)$$

dans laquelle G représente un dérivé fonctionnel d'un groupe carboxy, puis on le transforme éventuellement en un sel, les symboles $R^1$, A et n dans les formules I à V ayant les significations indiquées dans la revendication 1.

11. Procédé selon la revendication 10 pour la préparation d'acides acyl-biphénylylamino-alcanoique de formule générale I*:

$$R^{1*}—CO—N(A^*)—C_{n*}H_{2n*}—COOH \quad (I^*)$$

ainsi que de leurs sels avec des bases organiques ou inorganiques, caractérisé en ce que:

a) on acyle un acide biphénylylamino-alcanoïque de formule générale II*:

$$A^*—NH—C_{n*}H_{2n*}—COOH \quad (II^*)$$

en protégeant éventuellement le groupe carboxy, avec un dérivé acyle de formule générale III*:

$$R^{1*}—CO—R^{7*} \quad (III^*)$$

dans laquelle $R^{7*}$ représente un groupe qui s'éloigne ou un groupe $R^{1*}—CO—O—$, puis on le transforme éventuellement en un sel, ou

b) on hydrogène un acide biphénylylamino-alcénoïque de formule générale IV*:

$$R^{1*}\text{—CO—}\underset{\displaystyle A^{*}}{\underset{|}{N}}\text{—}C_{n*}H_{2n*-2}\text{—COOH} \qquad (IV^*)$$

en protégeant éventuellement le groupe carboxy, puis on le transforme éventuellement en un sel, ou

c) on solvolyse un dérivé fonctionnel d'un acide acyl-biphénylylamino-alcanoïque de formule générale V*

$$R^{1*}\text{—CO—}\underset{\displaystyle A^{*}}{\underset{|}{N}}\text{—}C_{n*}H_{2n*}\text{—}G^{*} \qquad (V^*)$$

dans laquelle G* représente un dérivé fonctionnel d'un groupe carboxy, puis on le transforme éventuellement en un sel, les symboles R1*, A* et n* dans les formules I* à V* ayant les significations indiquées dans la revendication 2.

12. Procédé selon la revendication 10 pour la préparation d'acides acyl-biphénylylamino-alcanoiques de formule générale I**:

$$R^{1**}\text{—CO—}\underset{\displaystyle A^{**}}{\underset{|}{N}}\text{—}C_{n**}H_{2n**}\text{—COOH} \qquad (I^{**})$$

ainsi que de leurs sels avec des bases organiques ou inorganiques, caractérisé en ce que:

a) on acyle un acide biphénylylamino-alcanoïque de formule générale II**:

$$A^{**}\text{—NH—}C_{n**}H_{2n**}\text{—COOH} \qquad (II^{**})$$

en protégeant éventuellement le groupe carboxy, avec un dérivé acyle de formule générale III**:

$$R^{1**}\text{—CO—}R^{7**} \qquad (III^{**})$$

dans laquelle R7** représente un groupe qui s'éloigne ou un groupe $R^{1**}$—CO—O—, puis on le transforme éventuellement en un sel, ou

b) on hydrogène un acide biphénylylamino-alcénoïque de formule générale IV**:

$$R^{1**}\text{—CO—}\underset{\displaystyle A^{**}}{\underset{|}{N}}\text{—}C_{n**}H_{2n**-2}\text{—COOH} \qquad (IV^{**})$$

en protégeant éventuellement le groupe carboxy, puis on le transforme éventuellement en un sel, ou

c) on solvolyse un dérivé fonctionnel d'un acide acyl-biphénylylamino-alcanoïque de formule générale V**:

$$R^{1**}\text{—CO—}\underset{\displaystyle A^{**}}{\underset{|}{N}}\text{—}C_{n**}H_{2n**}\text{—}G^{**} \qquad (V^{**})$$

dans laquelle G** représente un dérivé fonctionnel d'un groupe carboxy, puis on le transforme éventuellement en un sel, les symboles R1**, A** et n** dans les formules I** à V** ayant les significations indiquées dans la revendication 3.

13. Procédé selon la revendication 10 pour la préparation d'acides acyl-biphénylylamino-alcanoiques de formule générale I***:

$$R^{1***}\text{—CO—}\underset{\displaystyle A^{***}}{\underset{|}{N}}\text{—}C_{n***}H_{2n***}\text{—COOH} \qquad (I^{***})$$

ainsi que de leurs sels avec des bases organiques et inorganiques, caractérisé en ce que;

a) on acyle un acide biphénylylamino-alcanoïque de formule générale II***:

$$A^{***}—NH—C_{n^{***}}H_{2n^{***}}—COOH \qquad (II^{***})$$

en protégeant éventuellement le groupe carboxy, avec un dérivé acyle de formule générale III***:

$$R^{1***}—CO—R^{7***} \qquad (III^{***})$$

dans laquelle $R^{7***}$ représente un groupe qui s'éloigne ou un groupe $R^{1***}$—CO—O—, puis on le transforme éventuellement en un sel, ou

b) on hydrogène un acide biphénylylamino-alcénoïque de formule générale IV***:

$$R^{1***}—CO—N(A^{***})—C_{n^{***}}H_{2n^{***}-2}—COOH \qquad (IV^{***})$$

en protégeant éventuellement le groupe carboxy puis on le transforme éventuellement en un sel, ou

c) on solvolyse un dérivé fonctionnel d'un acide acyl-biphénylylamino-alcanoïque de formule générale V***:

$$R^{1***}—CO—N(A^{***})—C_{n^{***}}H_{2n^{***}}—G^{***} \qquad (V^{***})$$

dans laquelle G*** représente un dérivé fonctionnel d'un groupe carboxy, puis on le transforme éventuellement en un sel, les symboles $R^{1***}$, A*** et n*** dans les formules I*** à V*** ayant les significations indiquées dans la revendication 4.

14. Procédé selon la revendication 10 pour la préparation d'acides acyl-biphénylylamino-alcanoiques de formule générale I****:

$$R^{1****}—CO—N(A^{****})—C_{n^{****}}H_{2n^{****}}—COOH \qquad (I^{****})$$

ainsi que de leurs sels pharmacologiquement acceptables avec des bases organiques et inorganiques, caractérisé en ce que:

a) on acyle un acide biphénylylamino-alcanoïque de formule générale II****:

$$A^{****}—NH—C_{n^{****}}H_{2n^{****}}—COOH \qquad (II^{****})$$

en protégeant éventuellement le groupe carboxy, avec un dérivé acyle de formule générale III****:

$$R^{1****}—CO—R^{7****} \qquad (III^{****})$$

dans laquelle $R^{7****}$ représente un groupe qui s'éloigne ou un groupe $R^{1****}$—CO—O—, puis on le transforme éventuellement en un sel, ou

b) on hydrogène un acide biphénylylamino-alcénoïque de formule générale IV****:

$$R^{1****}—CO—N(A^{****})—C_{n^{****}}H_{2n^{****}-2}—COOH \qquad (IV^{****})$$

en protégeant éventuellement le groupe carboxy, puis on le transforme éventuellement en un sel, ou

c) on solvolyse un dérivé fonctionnel d'un acide acyl-biphénylylamino-alcanoïque de formule générale V****:

$$R^{1****}—CO—N(A^{****})—C_{n^{****}}H_{2n^{****}}—G^{****} \qquad (V^{****})$$

dans laquelle G**** représente un dérivé fonctionnel d'un groupe carboxy, puis on le transforme

éventuellement en un sel, les symboles R[1****], A**** et n**** dans les formules I**** et V**** ayant les significations indiquées dans la revendication 5.

15. Composés selon une ou plusieurs des revendications 1 à 8 pour le traitement et la prophylaxie de maladies dues à des affections de l'estomac ou de l'intestine ou encore à un rendement moins bon du pancréas, de la bile et/ou de foie.

**Revendications pour l'Etat contractant: AT**

1. Procédé de préparation d'acides acyl-biphénylylamino-alcanoiques de formule générale I:

$$R^1—CO—N(A)—C_nH_{2n}—COOH \qquad (I)$$

dans laquelle

$R^1$ représente un radical d'hydrocarbure aliphatique ou alicyclique ou un group phényle

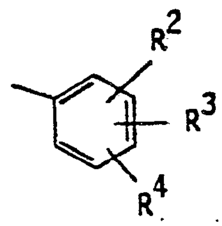

A représente un groupe biphénylyle éventuellement hydrogéné et éventuellement substitué par des atomes d'halogène, des groupes alkyle ou alcoxy contenant 1 à 4 atomes de carbone, des groupes amino éventuellement substitués, des groupes hydroxy ou des groupes nitro,

n est un nombre positif entier d'une valeur comprise entre 3 et 5,

$R^2$, $R^3$ et $R^4$ sont identiques ou différents et représentent un atome d'hydrogène, un atome d'halogène, un groupe alkyle, un groupe hydroxy, un groupe alcoxy, un groupe acyloxy, un groupe amino éventuellement substitué, un groupe nitro ou un groupe trifluorométhyle,

ainsi que de leurs sels avec des bases organiques ou inorganiques, caractérisé en ce que:

a) on acyle un acide biphénylylamino-alcanoïque de formule générale II:

$$A—NH—C_nH_{2n}—COOH \qquad (II)$$

dans laquelle A et n ont les significations indiquées ci-dessus, en protégeant éventuellement le groupe carboxy, avec un dérivé acyle de formule générale III:

$$R^1—CO—R^7 \qquad (III)$$

dans laquelle $R^7$ représente un groupe qui s'éloigne ou un groupe $R^1—CO—O—$, $R^1$ ayant la signification indiquée ci-dessus, puis on le transforme éventuellement en un sel, ou

b) on hydrogène un acide biphénylylamino-alcénoïque de formule générale IV:

$$R^1—CO—N(A)—C_nH_{2n-2}—COOH \qquad (IV)$$

dans laquelle $R^1$, A et n ont les significations indiquées ci-dessus, en protégeant éventuellement le groupe carboxy, puis on le transforme éventuellement en un sel, ou

c) on solvolyse un dérivé fonctionnel d'un acide acylbiphénylylamino-alcanoïque de formule générale V:

$$R^1—CO—N(A)—C_nH_{2n}—G \qquad (V)$$

dans laquelle $R^1$, A et n ont les significations indiquées ci-dessus, tandis que G représente un dérivé fonctionnel d'un groupe carboxy, puis on le transforme éventuellement en un sel.

2. Procédé selon la revendication 1 pour la préparation d'acides acyl-biphenylylamino-alcanoiques de formule générale I*:

$$R^{1*}—CO—N(A^*)—C_{n*}H_{2n*}—COOH \quad (I^*)$$

dans laquelle

$R^{1*}$ représente un radical d'hydrocarbure aliphatique contenant 1 à 7 atomes de carbone, un radical d'hydrocarbure alicyclique contenant 3 à 10 atomes de carbone ou un groupe phényle

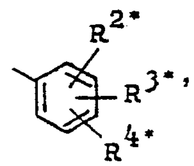

A* représente un groupe de formule:

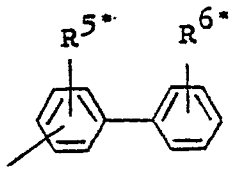 , 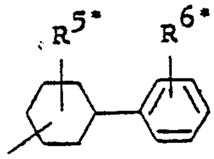 ou 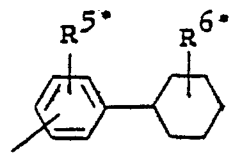

n* est un nombre positif entier d'une valeur comprise entre 3 et 5,

$R^{2*}$, $R^{3*}$ et $R^{4*}$ sont identiques ou différents et représentent chacun un atome d'hydrogène, un atome d'halogène, un groupe hydroxy, un groupe alkyle contenant 1 à 4 atomes de carbone, un groupe alcoxy contenant 1 à 4 atomes de carbone, un groupe alcanoyloxy contenant 2 à 5 atomes de carbone, un groupe nitro ou un groupe trifluorométhyle,

$R^{5*}$ et $R^{6*}$ sont identiques ou différents et représentent chacun un atome d'hydrogène, un atome d'halogène, un groupe méthyle, un groupe méthoxy, un groupe hydroxy ou un groupe nitro,

ainsi que de leurs sels avec des bases organiques ou inorganiques, caractérisé en ce que:

a) on acyle un acide biphénylylamino-alcanoïque de formule générale II*:

$$A^*—NH—C_{n*}H_{2n*}—COOH \quad (II^*)$$

dans laquelle A* et n* ont les significations indiquées ci-dessus, en protégeant éventuellement le groupe carboxy, avec un dérivé acyle de formule générale III*:

$$R^{1*}—CO—R^{7*} \quad (III^*)$$

dans laquelle $R^{7*}$ représente un groupe qui s'éloigne ou un groupe $R^{1*}—CO—O—$ et $R^{1*}$ a la signification indiquée ci-dessus, puis on le transforme éventuellement en un sel, ou

b) on hydrogène un acide biphénylylamino-alcénoïque de formule générale IV*:

$$R^{1*}—CO—N(A^*)—C_{n*}H_{2n*-2}—COOH \quad (IV^*)$$

dans laquelle $R^{1*}$, A* et n* ont les significations indiquées ci-dessus, en protégeant éventuellement le groupe carboxy, puis on le transforme éventuellement en un sel, ou

c) on solvolyse un dérivé fonctionnel d'un acide acyl-biphénylylamino-alcanoïque de formule générale V*

$$R^{1*}—CO—N(A^*)—C_{n*}H_{2n*}—G^* \quad (V^*)$$

dans laquelle $R^{1*}$, $A^*$ et $n^*$ ont les significations indiquées ci-dessus et $G^*$ représente un dérivé fonctionnel d'un groupe carboxy, puis on le transforme éventuellement en un sel.

3. Procédé selon la revendication 1 pour la préparation d'acides acyl-biphénylylamino-alcanoiques de formule générale:

$$R^{1**}—CO—\underset{\displaystyle A^{**}}{\underset{|}{N}}—C_{n**}H_{2n**}—COOH \qquad (I^{**})$$

dans laquelle

$R^{1**}$ représente un radical d'hydrocarbure aliphatique contenant 1 à 5 atomes de carbone, un radical d'hydrocarbure alicyclique contenant 5 à 7 atomes de carbone, ou un groupe phényle

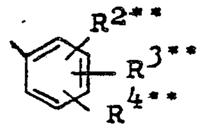

$A^{**}$ représente un groupe de formule:

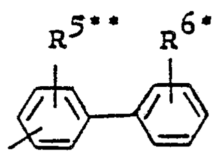

ou

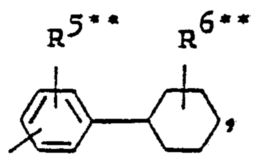

$n^{**}$ est un nombre positif entier d'une valeur comprise entre 3 et 5,

$R^{2**}$ représente un atome d'hydrogène,

$R^{3**}$ et $R^{4**}$ sont identiques ou différents et représentent chacun un atome d'hydrogène, un atome d'halogène, un groupe hydroxy, un groupe méthoxy, un groupe méthyle, un groupe alcanoyloxy contenant 2 à 5 atomes de carbone, un groupe nitro ou un groupe trifluorométhyle,

un des substituants $R^{5**}$ et $R^{6**}$ est un atome d'hydrogène, tandis que l'autre est un atome d'hydrogène, un atome d'halogène, un groupe méthyle, un groupe méthoxy, un groupe hydroxy ou un groupe nitro,

ainsi que de leurs sels avec des bases organiques ou inorganiques, caractérisé en ce que:

a) on acyle un acide biphénylylamino-alcanoïque de formule générale $II^{**}$:

$$A^{**}—NH—C_{n**}H_{2n**}—COOH \qquad (II^{**})$$

dans laquelle $A^{**}$ et $n^{**}$ ont les significations indiquées ci-dessus, en protégeant éventuellement le groupe carboxy, avec un dérivé acyle de formule générale $III^{**}$:

$$R^{1**}—CO—R^{7**} \qquad (III^{**})$$

dans laquelle $R^{7**}$ représente un groupe qui s'éloigne ou un groupe $R^{1**}—CO—O—$ et $R^{1**}$ a la signification indiquée ci-dessus, puis on le transforme éventuellement en un sel, ou

b) on hydrogène un acide biphénylylamino-alcénoïque de formule générale $IV^{**}$:

$$R^{1**}—CO—\underset{\displaystyle A^{**}}{\underset{|}{N}}—C_{n**}H_{2n**-2}—COOH \qquad (IV^{**})$$

dans laquelle $R^{1**}$, $A^{**}$ et $n^{**}$ ont les significations indiquées ci-dessus, en protégeant éventuellement le groupe carboxy, puis on le transforme éventuellement en un sel, ou

c) on solvolyse un dérivé fonctionnel d'un acide acyl-biphénylylamino-alcanoïque de formule générale $V^{**}$:

$$R^{1**}—CO—\underset{\displaystyle A^{**}}{\underset{|}{N}}—C_{n**}H_{2n**}—G^{**} \qquad (V^{**})$$

dans laquelle $R^{1**}$, $A^{**}$ et $n^{**}$ ont les significations indiquées ci-dessus et $G^{**}$ représente un dérivé fonctionnel d'un groupe carboxy, puis on le transforme éventuellement en un sel.

4. Procédé selon la revendication 1 pour la préparation d'acides acyl-biphénylylamino-alcanoiques de formule générale $I^{***}$:

$$R^{1***}—CO—N(A^{***})—C_{n^{***}}H_{2n^{***}}—COOH \qquad (I^{***})$$

dans laquelle

$R^{1***}$ représente un radical d'hydrocarbure aliphatique contenant 1 à 3 atomes de carbone ou un groupe phényle

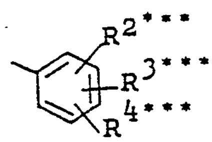

,

$A^{***}$ représente un groupe de formule:

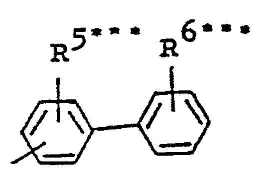

ou

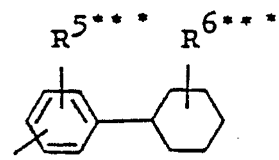

$n^{***}$ est un nombre positif entier d'une valeur comprise entre 3 et 5,

$R^{2***}$ représente un atome d'hydrogène,

$R^{3***}$ et $R^{4***}$ sont identiques ou différents et représentent chacun un atome d'hydrogène, un atome de fluor, un atome de chlore, un groupe hydroxy, un groupe méthoxy ou un groupe trifluorométhyle,

un des substituants $R^{5***}$ et $R^{6***}$ est un atome d'hydrogène, tandis que l'autre est un atome d'hydrogène ou un groupe méthoxy,

ainsi que de leurs sels avec des bases organiques et inorganiques, caractérisé en ce que:

a) on acyle un acide biphénylylamino-alcanoïque de formule générale $II^{***}$:

$$A^{***}—NH—C_{n^{***}}H_{2n^{***}}—COOH \qquad (II^{***})$$

dans laquelle $A^{***}$ et $n^{***}$ ont les significations indiquées ci-dessus, en protégeant éventuellement le groupe carboxy, avec un dérivé acyle de formule générale $III^{***}$:

$$R^{1***}—CO—R^{7***} \qquad (III^{***})$$

dans laquelle $R^{7***}$ représente un groupe qui s'éloigne ou un groupe $R^{1***}—CO—O—$ et $R^{1***}$ a la signification indiquée ci-dessus, puis on le transforme éventuellement en un sel, ou

b) on hydrogène un acide biphénylylamino-alcénoïque de formule générale $IV^{***}$:

$$R^{1***}—CO—N(A^{***})—C_{n^{***}}H_{2n^{***}-2}—COOH \qquad (IV^{***})$$

dans laquelle $R^{1***}$, $A^{***}$ et $n^{***}$ ont les significations indiquées ci-dessus, en protégeant éventuellement le groupe carboxy, puis on le transforme éventuellement en un sel, ou

c) on solvolyse un dérivé fonctionnel d'un acide acyl-biphénylylamino-alcanoïque de formule générale $V^{***}$:

$$R^{1***}—CO—N(A^{***})—C_{n^{***}}H_{2n^{***}}—G^{***} \qquad (V^{***})$$

dans laquelle $R^{1***}$, $A^{***}$ et $n^{***}$ ont les significations indiquées ci-dessus et $G^{***}$ représente un dérivé fonctionnel d'un groupe carboxy, puis on le transforme éventuellement en un sel.

5. Procédé selon la revendication 1 pour la préparation d'acides acyl-biphénylylamino-alcanoiques de formule générale $I^{****}$:

$$R^{1****}—CO—N(A^{****})—C_{n^{****}}H_{2n^{****}}—COOH \qquad (I^{****})$$

dans laquelle

$R^{1****}$ représente un groupe alkyle contenant 1 à 3 atomes de carbone, un groupe alcényle contenant 2 ou 3 atomes de carbone, ou un groupe phényle

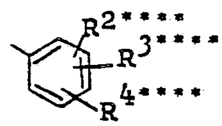

$A^{****}$ représente un groupe de formule:

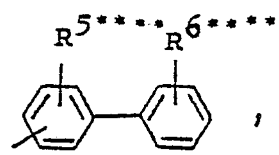
,

$n^{****}$ est un nombre positif entier d'une valeur comprise entre 3 et 5,

$R^{2****}$ représente un atome d'hydrogène,

$R^{3****}$ représente un atome d'hydrogène, un atome de fluor, un atome de chlore, un groupe hydroxy, un groupe méthoxy ou un groupe trifluorométhyl,

$R^{4****}$ représente un atome d'hydrogène ou un atome de chlore,

un des substituants $R^{5****}$ et $R^{6****}$ représente un atome d'hydrogène, tandis que l'autre représente un atome d'hydrogène ou un groupe méthoxy,

ainsi que de leurs sels pharmacologiquement acceptables avec des bases organiques et inorganiques, caractérisé en ce que:

a) on acyle un acide biphénylylamino-alcanoïque de formule générale $II^{****}$:

$$A^{****}—NH—C_{n^{****}}H_{2n^{****}}—COOH \qquad (II^{****})$$

dans laquelle $A^{****}$ et $n^{****}$ ont les significations indiquées ci-dessus, en protégeant éventuellement le groupe carboxy, avec un dérivé acyle de formule générale $III^{***}$:

$$R^{1****}—CO—R^{7****} \qquad (III^{****})$$

dans laquelle $R^{7****}$ représente un groupe qui s'éloigne ou un groupe $R^{1****}—CO—O—$, tandis que $R^{1****}$ a la signification indiquée ci-dessus, puis on le transforme éventuellement en un sel, ou

b) on hydrogène un acide biphénylylamino-alcénoïque de formule générale $IV^{****}$:

$$R^{1****}—CO—N(A^{****})—C_{n^{****}}H_{2n^{****}-2}—COOH \qquad (IV^{****})$$

dans laquelle $R^{1****}$, $A^{****}$ et $n^{****}$ ont les significations indiquées ci-dessus, en protégeant éventuellement le groupe carboxy, puis on le transforme éventuellement en un sel, ou

c) on solvolyse un dérivé fonctionnel d'un acide acyl-biphénylylamino-alcanoïque de formule générale $V^{****}$:

$$R^{1****}—CO—N(A^{****})—C_{n^{****}}H_{2n^{****}}—G^{****} \qquad (V^{****})$$

dans laquelle $R^{1****}$, $A^{****}$ et $n^{****}$ ont les significations indiquées ci-dessus, tandis que $G^{****}$ représente un dérivé fonctionnel d'un groupe carboxy, puis on le transforme éventuellement en un sel.

6. Procédé selon les revendications 4 et 5, $A^{***}$ ou $A^{****}$ représentant un radical 2-biphénylyle.

7. Procédé selon la revendication 5, $R^{3****}$ représentant un atome de fluor, un atome de chlore, un groupe hydroxy ou un groupe méthoxy et $R^{5****}$ et $R^{6****}$ chacun représentant un atome d'hydrogène.

8. Procédé selon la revendication 5, $A^{****}$ représentant un radical 2-biphénylyle, $R^{3****}$ représentant un atome de fluor, un atome de chlore, un groupe hydroxy ou un groupe méthoxy et $R^{5****}$ et $R^{6****}$ chacun représentant un atome d'hydrogène.

9. Procédé selon l'une ou plusieurs des revendications 1 à 8, où le groupe $R^7$, $R^{7*}$, $R^{7**}$, $R^{7***}$ ou $R^{7****}$ est un atome de chlore ou un atome de brome, et où G, G*, G**, G*** ou G**** est un groupe nitrile, un ester d'acide carboxylique ou un amide d'acide carboxylique.

**Claims for the Contracting States: BE, CH, DE, FR, GB, IT, LU, NL, SE**

1. Acylbiphenylylaminoalkanoic acids of the general formula I

$$R^1—CO—N(A)—C_nH_{2n}—COOH \quad (I)$$

in which

$R^1$ signifies an aliphatic or alicyclic hydrocarbon radical or a phenyl group

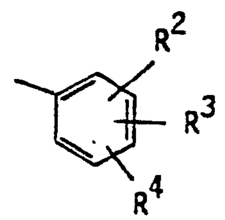

A signifies an optionally hydrogenated biphenylyl radical which may be substituted by halogen atoms, alkyl or alkoxy groups with 1 to 4 carbon atoms, optionally substituted amino groups, hydroxy or nitro groups,

n signifies a positive whole number from 3 to 5,

$R^2$, $R^3$ and $R^4$ are the same or different and signify a hydrogen atom, a halogen atom, an alkyl group, a hydroxy group, an alkoxy group, an acyloxy group, an optionally substituted amino group, a nitro group or a trifluoromethyl group,

and their salts of inorganic or organic bases.

2. Acylbiphenylylaminoalkanoic acids in accordance with Claim 1, characterized by the general formula I*

$$R^{1*}—CO—N(A^*)—C_{n*}H_{2n*}—COOH \quad (I^*),$$

in which

$R^{1*}$ signifies an aliphatic hydrocarbon radical with 1 to 7 carbon atoms, an alicyclic hydrocarbon radical with 3 to 10 carbon atoms or a phenyl radical

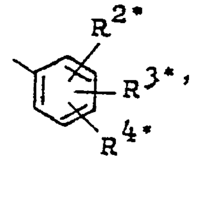

A* signifies a group of the formula

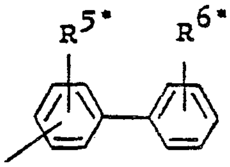 , 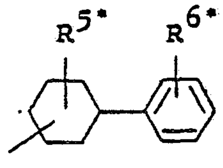 or 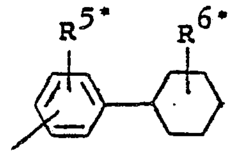

$n^*$ signifies a positive whole number from 3 to 5,

$R^{2*}$, $R^{3*}$ and $R^{4*}$ are the same or different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, a nitro group or a trifluoromethyl group,

$R^{5*}$ and $R^{6*}$ are the same or different and signify a hydrogen atom, a halogen atom, a methyl group, a methoxy group, a hydroxy group or a nitro group,
and their salts of inorganic or organic bases.

3. Acylbiphenylylaminoalkanoic acids in accordance with Claim 1, characterized by the general formula $I^{**}$

$$R^{1**}-CO-\underset{\displaystyle A^{**}}{\underset{|}{N}}-C_{n^{**}}H_{2n^{**}}-COOH \qquad (I^{**}),$$

in which

$R^{1**}$ signifies an aliphatic hydrocarbon radical with 1 to 5 carbon atoms, an alicyclic hydrocarbon radical with 5 to 7 carbon atoms or a phenyl radical

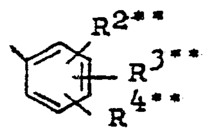

$A^{**}$ signifies a group of the formula

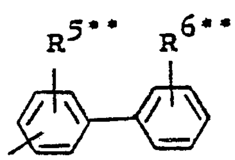 or 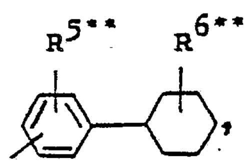

$n^{**}$ signifies a positive whole number from 3 to 5,

$R^{2**}$ signifies a hydrogen atom,

$R^{3**}$ and $R^{4**}$ are the same or different and signify a hydrogen atom, a halogen atom, a hydroxy group, a methoxy group, a methyl group an alkanoyloxy group with 2 to 5 carbon atoms, a nitro group or a trifluoromethyl group,

one of the substituents $R^{5**}$ or $R^{6**}$ signifies a hydrogen atom and the other signifies a hydrogen atom, a halogen atom, a methyl group, a methoxy group, a hydroxy group or a nitro group,
and their salts of inorganic or organic bases.

4. Acylbiphenylylaminoalkanoic acids in accordance with Claim 1, characterized by the general formula $I^{***}$

$$R^{1***}-CO-\underset{\displaystyle A^{***}}{\underset{|}{N}}-C_{n^{***}}H_{2n^{***}}-COOH \qquad (I^{***}),$$

in which

$R^{1***}$ signifies an aliphatic hydrocarbon radical with 1 to 3 carbon atoms or a phenyl radical

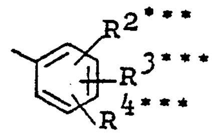,

A*** signifies a group of the formula

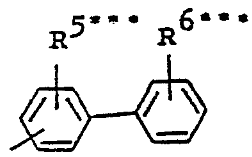 or 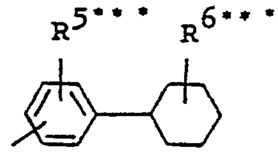

n*** is a positive whole number from 3 to 5,

$R^{2***}$ signifies a hydrogen atom,

$R^{3***}$ and $R^{4***}$ are the same or different and signify a hydrogen atom, a fluorine atom, a chlorine atom, a hydroxy group, a methoxy group or a trifluoromethyl group,

one of the substituents $R^{5***}$ or $R^{6***}$ signifies a hydrogen atom and the other signifies a hydrogen atom or a methoxy group,

and their salts of inorganic or organic bases.

5. Acylbiphenylylaminoalkanoic acids in accordance with Claim 1, characterized by the general formula I****

$$R^{1****}—CO—\underset{\underset{\displaystyle A^{****}}{|}}{N}—C_{n****}H_{2n****}—COOH \qquad (I^{****}),$$

in which

$R^{1****}$ signifies an alkyl group with 1 to 3 carbon atoms, an alkenyl radical with 2 or 3 carbon atoms, or a phenyl group

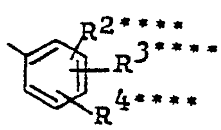

A**** signifies a group of the formula

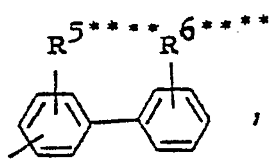,

n**** signifies a positive whole number from 3 to 5,

$R^{2****}$ signifies a hydrogen atom,

$R^{3****}$ signifies a hydrogen atom, a fluorine atom, a chlorine atom, a hydroxy group, a methoxy group or a trifluoromethyl group,

$R^{4****}$ signifies a hydrogen atom or a chlorine atom,

one of the substituents $R^{5****}$ or $R^{6****}$ signifies a hydrogen atom and the other signifies a hydrogen atom or a methoxy group,

and their pharmacologically compatible salts of inorganic or organic bases.

6. Compounds in accordance with Claim 4 or 5, in which A*** or A**** has the meaning of a 2-biphenylyl radical, and their pharmacologically compatible salts or inorganic or organic bases.

7. Compounds in accordance with Claim 5, in which $R^{3****}$ signifies a fluorine atom, a chlorine atom, a hydroxy group or a methoxy group and $R^{5****}$ and $R^{6****}$ denote hydrogen atoms, and their pharmacologically compatible salts of inorganic or organic bases.

8. Compounds in accordance with Claim 5, in which A**** has the meaning of a 2-biphenylyl radical, $R^{3****}$ signifies a fluorine atom, a chlorine atom, a hydroxy group or a methoxy group and $R^{5****}$ and $R^{6****}$ denote hydrogen atoms, and their pharmacologically compatible salts or inorganic or organic bases.

9. Pharmaceutical products containing one or more compounds in accordance with one or more of Claims 1 to 8 and/or their pharmacologically compatible salts of inorganic or organic bases.

10. Process for the production of acylbiphenylylaminoalkanoic acids of the general formula I

$$R^1{-}CO{-}\underset{\displaystyle A}{\underset{|}{N}}{-}C_nH_{2n}{-}COOH \qquad (I),$$

and their salts of inorganic or organic bases, characterized by the fact that

(a) a biphenylylaminoalkanoic acid of the general formula II

$$A{-}NH{-}C_nH_{2n}{-}COOH \qquad (II),$$

optionally with the protection of the carboxyl group, is acylated with an acyl derivative of the general formula III

$$R^1{-}CO{-}R^7 \qquad (III),$$

in which $R^7$ is a leaving group or a $R^1{-}CO{-}O{-}$ group, and if desired is then converted into a salt or

(b) a biphenylylaminoalkenoic acid of the general formula IV

$$R^1{-}CO{-}\underset{\displaystyle A}{\underset{|}{N}}{-}C_nH_{2n-2}{-}COOH \qquad (IV),$$

optionally with the protection of the carboxyl group, is hydrogenated and if desired is then converted into a salt, or

(c) a functional acylbiphenylylaminoalkanoic acid derivative of the general formula V

$$R^1{-}CO{-}\underset{\displaystyle A}{\underset{|}{N}}{-}C_nH_{2n}{-}G \qquad (V),$$

in which G signifies a functional derivative of a carboxyl group, is solvolysed and if desired is then converted into a salt, the symbols $R^1$, A and n in the formulae I to V having the meaning given in Claim 1.

11. Process in accordance with Claim 10 for the production of acylbiphenylylaminoalkanoic acids of the general formula I*

$$R^{1*}{-}CO{-}\underset{\displaystyle A^*}{\underset{|}{N}}{-}C_{n*}H_{2n*}{-}COOH \qquad (I^*),$$

and their salts of inorganic or organic bases, characterized by the fact that

(a) a biphenylylaminoalkanoic acid of the general formula II*

$$A^*{-}NH{-}C_{n*}H_{2n*}{-}COOH \qquad (II^*),$$

optionally with the protection of the carboxyl group, is acylated with an acyl derivative of the general formula III*

$$R^{1*}{-}CO{-}R^{7*} \qquad (III^*),$$

in which $R^{7*}$ is a leaving group or a $R^{1*}$—CO—O— group, and if desired is then converted into a salt or

(b) a biphenylylaminoalkenoic acid of the general formula IV*

$$R^{1*}\text{—CO—}\underset{\overset{|}{A^*}}{N}\text{—}C_{n*}H_{2n*-2}\text{—COOH} \qquad (IV^*),$$

optionally with the protection of the carboxyl group, is hydrogenated and if desired is then converted into a salt, or

(c) a functional acylbiphenylylaminoalkanoic acid derivative of the general formula V*

$$R^{1*}\text{—CO—}\underset{\overset{|}{A^*}}{N}\text{—}C_{n*}H_{2n*}\text{—}G^* \qquad (V^*),$$

in which G* signifies a functional derivative of a carboxyl group, is solvolysed and if desired is then converted into a salt, the symbols $R^{1*}$, A* and n* in the formulae I* to V* having the meaning given in Claim 2.

12. Process in accordance with Claim 10 for the production of acylbiphenylylaminoalkanoic acids of the general formula I**

$$R^{1**}\text{—CO—}\underset{\overset{|}{A^{**}}}{N}\text{—}C_{n**}H_{2n**}\text{—COOH} \qquad (I^{**}),$$

and their salts of inorganic or organic bases, characterized by the fact that

(a) a biphenylylaminoalkanoic acid of the general formula II**

$$A^{**}\text{—NH—}C_{n**}H_{2n**}\text{—COOH} \qquad (II^{**}),$$

optionally with the protection of the carboxyl group, is acylated with an acyl derivative of the general formula III**

$$R^{1**}\text{—CO—}R^{7**} \qquad (III^{**}),$$

in which $R^{7**}$ is a leaving group or a $R^{1**}$—CO—O group, and if desired is then converted into a salt or

(b) a biphenylylaminoalkanoic acid of the general formula IV**

$$R^{1**}\text{—CO—}\underset{\overset{|}{A^{**}}}{N}\text{—}C_{n**}H_{2n**-2}\text{—COOH} \qquad (IV^{**}),$$

optionally with the protection of the carboxyl group, is hydrogenated and if desired is then converted into a salt, or

(c) a functional acylbiphenylylaminoalkanoic acid derivative of the general formula V**

$$R^{1**}\text{—CO—}\underset{\overset{|}{A^{**}}}{N}\text{—}C_{n**}H_{2n**}\text{—}G^{**} \qquad (V^{**}),$$

in which G** signifies a functional derivative of a carboxyl group, is solvolysed and if desired is then converted into a salt, the symbols $R^{1**}$, A** and n** in the formulae I** to V** having the meaning given in Claim 3.

13. Process in accordance with Claim 10 for the production of acylbiphenylylaminoalkanoic acids of the general formula I***

$$R^{1***}—CO—N(A^{***})—C_{n***}H_{2n***}—COOH \qquad (I^{***}),$$

and their salts of inorganic or organic bases, characterized by the fact that

(a) a biphenylylaminoalkanoic acid of the general formula II***

$$A^{***}—NH—C_{n***}H_{2n***}—COOH \qquad (II^{***})$$

optionally with the protection of the carboxyl group, is acylated with an acyl derivative of the general formula III***

$$R^{1***}—CO—R^{7***} \qquad (III^{***}),$$

in which $R^{7***}$ is a leaving group or a $R^{1***}$—CO—O— group and if desired is then converted into a salt or

(b) a biphenylylaminoalkenoic acid of the general formula IV***

$$R^{1***}—CO—N(A)—C_{n***}H_{2n***-2}—COOH \qquad (IV^{***}),$$

optionally with the protection of the carboxyl group, is hydrogenated and if desired is then converted into a salt, or

(c) a functional acylbiphenylylaminoalkanoic acid derivative of the general formula V***

$$R^{1***}—CO—N(A^{***})—C_{n***}H_{2n***}—G^{***} \qquad (V^{***}),$$

in which G*** signifies a functional derivative of a carboxyl group, is solvolysed and if desired is then converted into a salt, the symbols $R^{1***}$, A*** and n*** in the formulae I*** to V*** having the meaning given in Claim 4.

14. Process in accordance with Claim 10 for the production of acylbiphenylylaminoalkanoic acids of the general formula I****

$$R^{1****}—CO—N(A^{****})—C_{n****}H_{2n****}—COOH \qquad (I^{****}),$$

and their salts of inorganic or organic bases, characterized by the fact that

(a) a biphenylylaminoalkanoic acid of the general formula II****

$$A^{****}—NH—C_{n****}H_{2n****}—COOH \qquad (II^{****}),$$

optionally with the protection of the carboxyl group, is acylated with an acyl derivative of the general formula III****

$$R^{1****}—CO—R^{7****} \qquad (III^{****}),$$

in which $R^{7****}$ is a leaving group or a $R^{1****}$—CO—O— group, and if desired is then converted into a salt or

(b) a biphenylylaminoalkenoic acid of the general formula IV****

$$R^{1****}—CO—N(A^{****})—C_{n****}H_{2n****-2}—COOH \qquad (IV^{****}),$$

optionally with the protection of the carboxyl group, is hydrogenated and if desired is then converted into a salt, or

(c) a functional acylbiphenylylaminoalkanoic acid derivative of the general formula V****

$$R^{1****}-CO-\underset{A^{****}}{\underset{|}{N}}-C_{n^{****}}H_{2n^{****}}-G^{****} \qquad (V^{****}),$$

in which G**** signifies a functional derivative of a carboxyl group, is solvolysed and if desired is then converted into a salt, the symbols $R^{1****}$, A**** and n**** in the formulae I**** to V**** having the same meaning given in Claim 5.

15. Compounds in accordance with one or more of the Claims 1 to 8 for the treatment and prophylaxis of diseases which are attributable to disorders of the stomach or intestine or to reduced performances of the pancreas, bile and/or liver.

**Claims for the Contracting State: AT**

1. Process for the production of acylbiphenylylaminoalkanoic acids of the general formula I

$$R^1-CO-\underset{A}{\underset{|}{N}}-C_nH_{2n}-COOH \qquad (I)$$

in which

$R^1$ signifies an aliphatic or alicyclic hydrocarbon radical or a phenyl group

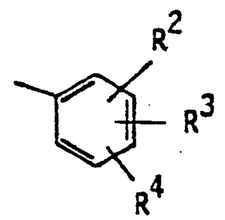

A signifies an optionally hydrogenated biphenylyl radical which may be substituted by halogen atoms, alkyl or alkoxy groups with 1 to 4 carbon atoms, optionally substituted amino groups, hydroxy or nitro groups,

n signifies a positive whole number from 3 to 5,

$R^2$, $R^3$ and $R^4$ are the same or different and signify a hydrogen atom, a halogen atom, an alkyl group, a hydroxy group, an alkoxy group, an acyloxy group, an optionally substituted amino group, a nitro group or a trifluoromethyl group,

and their salts of inorganic or organic bases, characterized by the fact that

(a) a biphenylylaminoalkanoic acid of the general formula II

$$A-NH-C_nH_{2n}-COOH \qquad (II),$$

in which A and n have the meanings given above, optionally with the protection of the carboxyl group, is acylated with an acyl derivative of the general formula III

$$R^1-CO-R^7 \qquad (III),$$

in which $R^7$ is a leaving group or a $R^1-CO-O-$ group, and $R^1$ has the meaning given above, and if desired is then converted into a salt or

(b) a biphenylylaminoalkenoic acid of the general formula IV

$$R^1-CO-\underset{A}{\underset{|}{N}}-C_nH_{2n-2}-COOH \qquad (IV),$$

in which $R^1$, A and n have the meanings given above, optionally with the protection of the carboxyl group, is hydrogenated and if desired is then converted into a salt, or

(c) a functional acylbiphenylylaminoalkanoic acid derivative of the general formula V

$$R^1\text{—}CO\text{—}\underset{\displaystyle A}{\underset{|}{N}}\text{—}C_nH_{2n}\text{—}G \qquad (V),$$

in which $R^1$, A and n have the meanings given above and G signifies a functional derivative of a carboxyl group, is solvolysed and if desired is then converted into a salt.

2. Process in accordance with Claim 1 for the production of acylbiphenylylaminoalkanoic acids of the general formula I*

$$R^{1*}\text{—}CO\text{—}\underset{\displaystyle A^*}{\underset{|}{N}}\text{—}C_{n*}H_{2n*}\text{—}COOH \qquad (I^*),$$

in which

$R^{1*}$ signifies an aliphatic hydrocarbon radical with 1 to 7 carbon atoms, an alicyclic hydrocarbon radical with 3 to 10 carbon atoms or a phenyl radical

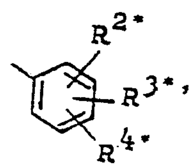

A* signifies a group of the formula

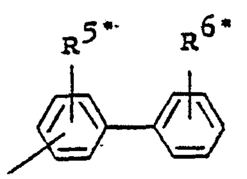
,
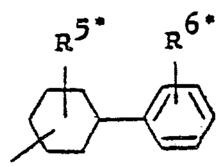
or
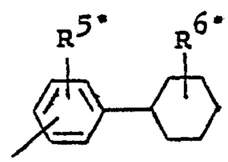

n* signifies a positive whole number from 3 to 5,

$R^{2*}$, $R^{3*}$ and $R^{4*}$ are the same or different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, a nitro group or a trifluoromethyl group,

$R^{5*}$ and $R^{6*}$ are the same or different and signify a hydrogen atom, a halogen atom, a methyl group, a methoxy group, a hydroxy group or a nitro group,
and their salts of inorganic or organic bases, characterized by the fact that

(a) a biphenylylaminoalkanoic acid of the general formula II*

$$A^*\text{—}NH\text{—}C_{n*}H_{2n*}\text{—}COOH \qquad (II^*),$$

in which A* and n* have the meanings given above, optionally with the protection of the carboxyl group, is acylated with an acyl derivative of the general formula III*

$$R^{1*}\text{—}CO\text{—}R^{7*} \qquad (III^*),$$

in which $R^{7*}$ is a leaving group or a $R^{1*}$—CO—O— group and $R^{1*}$ has the meaning given above, and if desired is then converted into a salt or

(b) a biphenylylaminoalkanoic acid of the general formula IV*

$$R^{1*}—CO—\underset{\displaystyle A^*}{\underset{|}{N}}—C_{n*}H_{2n*-2}—COOH \qquad (IV^*),$$

in which $R^{1*}$, A* and n* have the meanings given above, optionally with the protection of the carboxyl group, is hydrogenated and if desired is then converted into a salt, or

(c) a functional acylbiphenylylaminoalkanoic acid derivative of the general formula V*

$$R^{1*}—CO—\underset{\displaystyle A^*}{\underset{|}{N}}—C_{n*}H_{2n*}—G^* \qquad (V^*),$$

in which $R^{1*}$, A* and n* have the meanings given above and G* signifies a functional derivative of a carboxyl group, is solvolysed and if desired is then converted into a salt.

3. Process in accordance with Claim 1 for the production of acylbiphenylylaminoalkanoic acids of the general formula I**

$$R^{1**}—CO—\underset{\displaystyle A^{**}}{\underset{|}{N}}—C_{n**}H_{2n**}—COOH \qquad (I^{**}),$$

in which

$R^{1**}$ signifies an aliphatic hydrocarbon radical with 1 to 5 carbon atoms, an alicyclic hydrocarbon radical with 5 to 7 carbon atoms or a phenyl radical

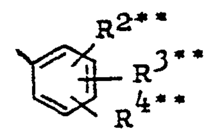

A** signifies a group of the formula

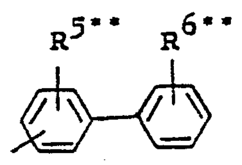

or

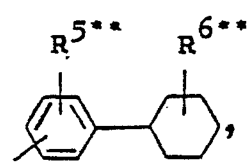

n** signifies a positive whole number from 3 to 5,

$R^{2**}$ signifies a hydrogen atom,

$R^{3**}$ and $R^{4**}$ are the same or different and signify a hydrogen atom, a halogen atom, a hydroxy group, a methoxy group, a methyl group, an alkanoyloxy group with to 5 carbon atoms, a nitro group or a trifluoromethyl group,

one of the substituents $R^{5**}$ or $R^{6**}$ signifies a hydrogen atom and the other signifies a hydrogen atom, a halogen atom, a methyl group, a methoxy group, a hydroxy group or a nitro group, and their salts of inorganic or organic bases, characterized by the fact that

(a) a biphenylylaminoalkanoic acid of the general formula II**

$$A^{**}—NH—C_{n**}H_{2n**}—COOH \qquad (II^{**}),$$

in which A** and n** have the meanings given above, optionally with the protection of the carboxyl group, is acylated with an acyl derivative of the general formula III**

$$R^{1**}—CO—R^{7**} \qquad (III^{**}),$$

in which $R^{7**}$ is a leaving group or a $R^{1**}—CO—O—$ group and $R^{1**}$ has the meaning given above, and if desired is then converted into a salt or

(b) a biphenylylaminoalkenoic acid of the general formula IV**

$$R^{1**}\text{—CO—}\underset{A^{**}}{\underset{|}{N}}\text{—}C_{n**}H_{2n**-2}\text{—COOH} \qquad (IV^{**}),$$

in which $R^{1**}$, A** and n** have the meanings given above, optionally with the protection of the carboxyl group, is hydrogenated and if desired is then converted into a salt, or

(c) a functional acylbiphenylylaminoalkanoic acid derivative of the general formula V**

$$R^{1**}\text{—CO—}\underset{A^{**}}{\underset{|}{N}}\text{—}C_{n**}H_{2n**}\text{—}G^{**} \qquad (V^{**}),$$

in which $R^{1**}$, A** and n** have the meanings given above and G** signifies a functional derivative of a carboxyl group, is solvolysed and if desired is then converted into a salt

4. Process in accordance with Claim 1 for the production of acylbiphenylylaminoalkanoic acids of the general formula I***

$$R^{1***}\text{—CO—}\underset{A^{***}}{\underset{|}{N}}\text{—}C_{n***}H_{2n***}\text{—COOH} \qquad (I^{***}),$$

in which

$R^{1***}$ signifies an aliphatic hydrocarbon radical with 1 to 3 carbon atoms or a phenyl radical

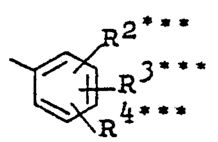

A*** signifies a group of the formula

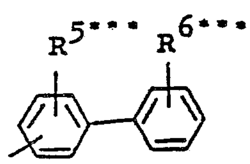

or

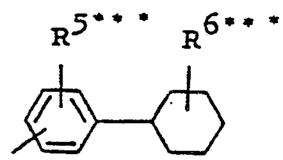

n*** is a positive whole number from 3 to 5,

$R^{2***}$ signifies a hydrogen atom,

$R^{3***}$ and $R^{4***}$ are the same or different and signify a hydrogen atom, a fluorine atom, a chlorine atom, a hydroxy group, a methoxy group or a trifluoromethyl group,

one of the substituents $R^{5***}$ or $R^{6***}$ signifies a hydrogen atom and the other signifies a hydrogen atom or a methoxy group,

and their salts of inorganic or organic bases, characterized by the fact that

(a) a biphenylylaminoalkanoic acid of the general formula II***

$$A^{***}\text{—NH—}C_{n***}H_{2n***}\text{—COOH} \qquad (II^{***}),$$

in which A*** and n*** have the meanings given above, optionally with the protection of the carboxyl group, is acylated with an acyl derivative of the general formula III***

$$R^{1***}\text{—CO—}R^{7***} \qquad (III^{***}),$$

in which $R^{7***}$ is a leaving group or a $R^{1***}$—CO—O— group and $R^{1***}$ has the meaning given above, and if desired is then converted into a salt or

(b) a biphenylylaminoalkanoic acid of the general formula IV***

$$R^{1***}-CO-\underset{\displaystyle A^{***}}{\underset{|}{N}}-C_{n***}H_{2n***-2}-COOH \qquad (IV^{***}),$$

in which $R^{1***}$, A*** and n*** have the meanings given above, optionally with the protection of the carboxyl group, is hydrogenated and if desired is then converted into a salt, or

(c) a functional acylbiphenylylaminoalkanoic acid derivative of the general formula V***

$$R^{1***}-CO-\underset{\displaystyle A^{***}}{\underset{|}{N}}-C_{n***}H_{2n***}-G^{***} \qquad (V^{***}),$$

in which $R^{1***}$, A*** and n*** have the meanings given above and G*** signifies a functional derivative of a carboxyl group, is solvolysed and if desired is then converted into a salt.

5. Process in accordance with Claim 1 for the production of acylbiphenylylaminoalkanoic acids of the general formula I****

$$R^{1****}-CO-\underset{\displaystyle A^{****}}{\underset{|}{N}}-C_{n****}H_{2n****}-COOH \qquad (I^{****}),$$

in which

$R^{1****}$ signifies an alkyl group with 1 to 3 carbon atoms, an alkenyl radical with 2 or 3 carbon atoms, or a phenyl group

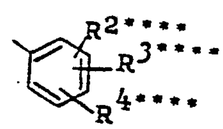

A**** signifies a group of the formula

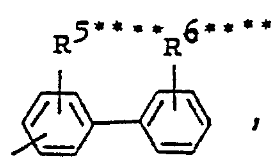
,

n**** signifies a positive whole number from 3 to 5,

$R^{2****}$ signifies a hydrogen atom,

$R^{3****}$ signifies a hydrogen atom, a fluorine atom, a chlorine atom, a hydroxy group, a methoxy group or a trifluoromethyl group,

$R^{4****}$ signifies a hydrogen atom or a chlorine atom,

one of the substituents $R^{5****}$ or $R^{6****}$ signifies a hydrogen atom and the other signifies a hydrogen atom or a methoxy group,

and their pharmacologically compatible salts of inorganic or organic bases, characterized by the fact that

(a) a biphenylylaminoalkanoic acid of the general formula II****

$$A^{****}-NH-C_{n****}H_{2n****}-COOH \qquad (II^{****}),$$

in which A**** and n**** have the meanings given above, optionally with the protection of the carboxyl group, is acylated with an acyl derivative of the general formula III****

$$R^{1****}-CO-R^{7****} \qquad (III^{****}),$$

in which $R^{7****}$ is a leaving group or a $R^{1****}$ —CO—O group, and $R^{1****}$ has the meaning given above, and if desired is then converted into a salt or

(b) a biphenylylaminoalkenoic acid of the general formula IV****

$$R^{1****}\text{—CO—}\underset{\underset{\textstyle A^{****}}{|}}{\text{N}}\text{—}C_{n^{****}}H_{2n^{****}-2}\text{—COOH} \qquad (IV^{****}),$$

in which $R^{1****}$, A**** and n**** have the meanings given above, optionally with the protection of the carboxyl group, is hydrogenated and if desired is then converted into a salt, or

(c) a functional acylbiphenylylaminoalkanoic acid derivative of the general formula V****

$$R^{1****}\text{—CO—}\underset{\underset{\textstyle A^{****}}{|}}{\text{N}}\text{—}C_{n^{****}}H_{2n^{****}}\text{—G —}G^{****} \qquad (V^{****}),$$

in which $R^{1****}$, A**** and n**** have the meanings given above and G**** signifies a functional derivative of a carboxyl group, is solvolysed and if desired is then converted into a salt.

6. A process in accordance with Claim 4 or 5, in which A*** or A**** has the meaning of a 2-biphenylyl radical.

7. Process in accordance with Claim 5, in which $R^{3****}$ signifies a fluorine atom, a chlorine atom, a hydroxy group or a methoxy group and $R^{5****}$ and $R^{6****}$ denote hydrogen atoms.

8. Process in accordance with Claim 5, in which A**** has the meaning of a 2-biphenylyl radical, $R^{3****}$ signifies a fluorine atom, a chlorine atom, a hydroxy group or a methoxy group and $R^{5****}$ and $R^{6****}$ denote hydrogen atoms.

9. Process in accordance with one or more of the Claims 1 to 8, wherein the leaving group $R^7$, $R^{7*}$ $R^{7**}$, $R^{7***}$ or $R^{7****}$ denotes a chlorine or bromine atom and G, G*, G**, G*** or G**** denotes a nitrile, a carboxylic acid ester or a carboxylic acid amide group.